(19) Europäisches Patentamt
European Patent Office
Office européen des brevets

(11) Veröffentlichungsnummer : **0 082 817**
**B1**

(12) # EUROPÄISCHE PATENTSCHRIFT

(45) Veröffentlichungstag der Patentschrift : **04.03.87**

(21) Anmeldenummer : **82810538.7**

(22) Anmeldetag : **13.12.82**

(51) Int. Cl.⁴ : **G 03 C 7/26**, C 07 D211/46, C 07 D211/58, C 07 D211/94, C 07 D211/22

(54) **Farbphotographisches Aufzeichnungsmaterial.**

(30) Priorität : **17.12.81 CH 8067/81**

(43) Veröffentlichungstag der Anmeldung : **29.06.83 Patentblatt 83/26**

(45) Bekanntmachung des Hinweises auf die Patenterteilung : **04.03.87 Patentblatt 87/10**

(84) Benannte Vertragsstaaten : **BE CH DE FR GB IT LI NL**

(56) Entgegenhaltungen :
**EP-A- 0 011 051**
**CH-A- 586 251**
**FR-A- 2 351 961**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Klybeckstrasse 141**
**CH-4002 Basel (CH)**

(72) Erfinder : **Leppard, David G., Dr.**
**Kohlplatzweg 26**
**CH-4310 Rheinfelden (CH)**
Erfinder : **Rody, Jean, Dr.**
**Rütiring 82**
**CH-4125 Riehen (CH)**

**EP 0 082 817 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein farbphotographisches Aufzeichnungsmaterial, das in mindestens einer lichtempfindlichen Silberhalogenidemulsionsschicht und/oder in mindestens einer der üblichen Hilfsschichten mindestens ein Polyalkylpiperidin-Lichtschutzmittel enthält.

Aus der FR-A 2 351 961 sind Stabilisatoren bekannt, die eine sterisch gehinderte, Hydroxybenzylgruppe und mindestens einen Polyalkylpiperidinrest enthalten. Eine Verwendung dieser Stabilisatoren für photographisches Material wird nicht vorgeschlagen.

In der EP-OS 11 051 ist bereits ein farbphotographisches Aufzeichnungsmaterial beschrieben, welches zur Verbesserung der Lichtechtheit der daraus erhaltenen Farbbilder und zur Unterdrückung einer unerwünschten Schleierbildung Polyalkylpiperidin-Lichtschutzmittel enthält.

Es wurde nun gefunden, dass überraschenderweise eine noch bessere Lichtschutzwirkung erreicht werden kann, wenn die nachfolgend beschriebenen Verbindungen der Formel I in das farbphotographische Aufzeichnungsmaterial eingearbeitet werden.

Gegenstand der vorliegenden Anmeldung ist demnach ein farbphotographisches Aufzeichnungsmaterial, das in mindestens einer lichtempfindlichen Silberhalogenidemulsionsschicht, einer Zwischenschicht und/oder einer Schutzschicht mindestens eine Polyalkylpiperidinverbindung als Lichtschutzmittel enthält, dadurch gekennzeichnet, dass die Polyalkylpiperidinverbindung der Formel I

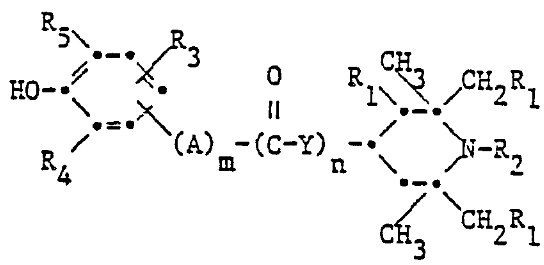
(I)

entspricht, worin

$R_1$ und $R_3$ unabhängig voneinander Wasserstoff oder Methyl sind

$R_2$ Hydroxy, $C_1$-$C_{12}$ Alkyl, $C_3$-$C_{12}$ Alkenyl, $C_3$-$C_4$ Alkinyl, 2-Hydroxyäthyl, $C_2$-$C_{11}$ Alkoxyalkyl, $C_7$-$C_{14}$ Aralkyl oder

eine Gruppe der Formel $-(CH_2)_p-CH(R_6)-X_1$ oder $-CH(R_6)-X_2$ ist, worin p die Zahlen 1, 2 oder 3 bedeutet, $X_1$ Halogen, Cyano, $-OR_7$, $-OC(O)R_7$, $-OC(O)N(R_7)(R_8)$, $-C(O)OR_7$, $-C(O)N(R_7)(R_8)$ ist, $X_2$ Halogen, Cyano, 1,2-Epoxyäthyl, $-C(O)OR_7$, $-C(O)N(R_7)(R_8)$ und $R_6$ Wasserstoff, Methyl oder Phenyl sind, wobei $R_7$ Wasserstoff, $C_1$-$C_{12}$ Alkyl, $C_2$-$C_{12}$ Alkenyl, $C_3$-$C_{12}$ Cycloalkyl, Phenyl, $C_7$-$C_{14}$ Alkaryl, $C_7$-$C_{14}$ Aralkyl und $R_8$ Wasserstoff oder $C_1$-$C_4$ Alkyl bedeuten oder $R_7$ und $R_8$ zusammen mit dem Stickstoffatom an dem sie gebunden sind einen 5- oder 6-gliedrigen Heterocyclus bilden, oder $R_2$ eine Gruppe der Formel II

$$L-CO- \qquad (II)$$

bedeutet, worin L Wasserstoff, $C_1$-$C_{12}$ Alkyl, $C_2$-$C_{12}$ Alkenyl, $C_3$-$C_4$ Alkinyl, $C_3$-$C_{12}$ Cycloalkyl, $C_7$-$C_{14}$ Alkaryl, $C_7$-$C_{14}$ Aralkyl, Chlormethyl, unsubstituiertes oder durch zwei $C_1$-$C_4$ Alkyl und ein Hydroxy substituiertes Phenyl oder eine Gruppe $-OR_9$ ist, worin $R_9$ $C_1$-$C_{12}$ Alkyl, Cyclohexyl, $C_2$-$C_{12}$ Alkenyl, Benzyl oder Phenyl bedeutet, oder L eine Gruppe der Formel III

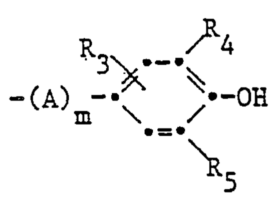
(III)

ist oder $R_2$ eine Gruppe der Formel IV

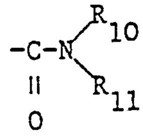
(IV)

bedeutet, worin $R_{10}$ und $R_{11}$ unabhängig voneinander $C_1$-$C_{12}$ Alkyl, $C_3$-$C_{12}$ Alkenyl, $C_3$-$C_{12}$ Cycloalkyl, Phenyl, $C_7$-$C_{14}$ Alkaryl, $C_7$-$C_{14}$ Aralkyl bedeuten und $R_{11}$ zusätzlich auch Wasserstoff sein kann, oder $R_2$ eine Gruppe der Formel V

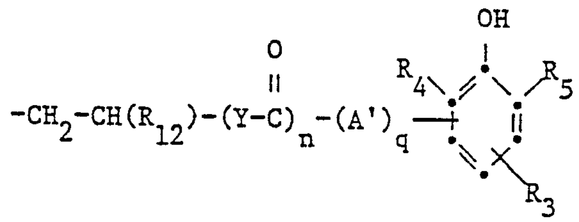

(V)

ist, worin A' Methylen oder eine Gruppe $-CH_2-CH(R_{13})-$ bedeutet und q Null oder 1 ist, und $R_{12}$ und $R_{13}$ unabhängig voneinander Wasserstoff, Methyl, Aethyl, Phenoxymethyl oder Phenyl sind, oder $R_2$ eine Gruppe der Formel VI

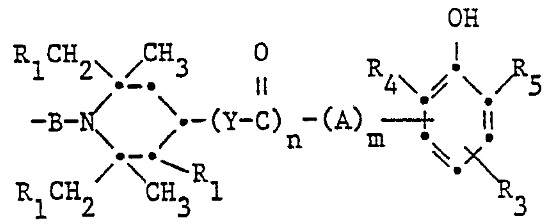

(VI)

ist, worin B eine Gruppe $C_rH_{2r}$ ist, in welcher r eine Zahl 2 bis 12 bedeutet, oder $C_4$-$C_8$ Alkenylen, $C_4$-$C_8$ Alkinylen, Phenylen, Xylylen, Bitolylen, $C_5$-$C_{12}$ Cycloalkylen oder eine Gruppe $-CONH-B_1-NHCO-$, worin $B_1$ eine Gruppe $C_rH_{2r}$, Phenylen, Naphthylen, Tolylen oder eine Gruppe der Formeln

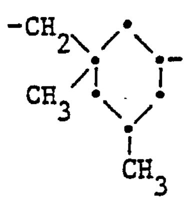

,

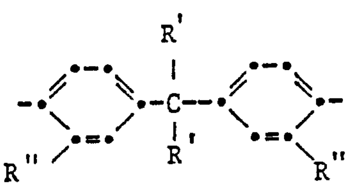

oder

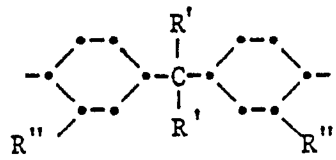

.

bedeutet, worin

R' Wasserstoff, Methyl oder Ethyl und

R'' Wasserstoff oder Methyl bedeuten und

r die obenangegebene Bedeutung hat,

oder $R_2$ eine der Gruppen $-S(O)_zR_{14}$ oder $-OR_{15}$ bedeutet, worin z die Zahl 1 oder 2 ist,

$R^{14}$ $C_1$-$C_{12}$ Alkyl, $C_7$-$C_{14}$ Alkaryl oder Phenyl ist und $R_{15}$ $C_1$-$C_{12}$ Alkyl, $C_3$-$C_{12}$ Alkenyl, $C_7$-$C_{14}$ Aralkyl oder eine Gruppe der Formel L'—CO— bedeutet, worin L' die oben für L angegebene Bedeutung hat,

$R_4$ und $R_5$ unabhängig voneinander $C_1$-$C_{12}$ Alkyl, $C_3$-$C_{12}$ Cycloalkyl, $C_7$-$C_{14}$ Aralkyl, $C_7$-$C_{14}$ Alkaryl oder Phenyl bedeuten und $R_4$ zusätzlich Wasserstoff oder eine Gruppe der Formel Ia

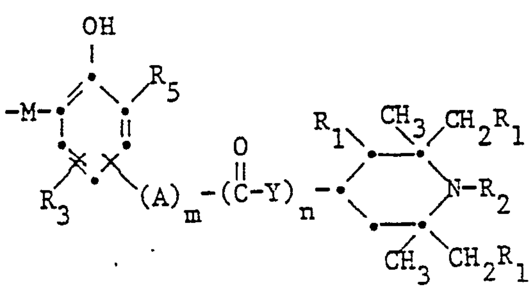

(Ia)

bedeutet, worin $R_1$, $R_2$, $R_3$, $R_5$, A, Y, m und n die angegebene Bedeutung haben und

M eine direkte Bindung, $-\overset{D_1}{\underset{D_2}{C}}-$, $-S-$, $-S-S-$, $-\overset{O}{\underset{O}{S}}-$, $-\overset{O}{S}-$, $-CH_2-S-CH_2-$, $-CH_2-O-CH_2-$,

—O—, —N($D_3$)— ist und $D_1$ und $D_2$ unabhängig voneinander Wasserstoff, $C_1$-$C_{18}$ Alkyl, durch 1 bis 3 —S— unterbrochenes Alkyl, Phenyl bedeuten oder $D_1$ und $D_2$ zusammen mit dem sie bindenden C-Atom einen 5- oder 6-gliedrigen aliphatischen Ring bilden und $D_3$ Wasserstoff, $C_1$-$C_{18}$ Alkyl oder Phenyl ist,

Y —O— oder —N($R_{16}$)— ist, worin $R_{16}$ Wasserstoff, $C_1$-$C_{18}$ Alkyl, $C_3$-$C_{12}$ Alkenyl, $C_3$-$C_{12}$ Cycloalkyl, Phenyl, $C_7$-$C_{14}$ Alkaryl, $C_7$-$C_{14}$ Aralkyl, $C_3$-$C_{11}$ Alkoxyalkyl, eine Gruppe der Formel VII

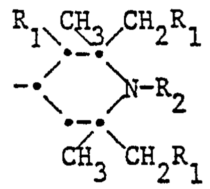

(VII)

oder eine Gruppe —Z—D ist, worin Z eine unsubstituierte oder durch eine Methylgruppe substituierte —$(CH_2)_s$-Gruppe ist, wobei s die Zahlen 2 bis 4 bedeutet und D Hydroxy, —$OR_{17}$ oder —N($R_{18}$)($R_{19}$) ist, worin $R_{17}$ und $R_{18}$ Wasserstoff, $C_1$-$C_{12}$ Alkyl, $C_3$-$C_{12}$ Cycloalkyl oder eine Gruppe der Formel VIII

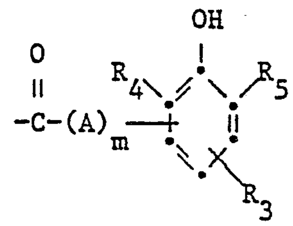

(VIII)

sind und $R_{17}$ zusätzlich auch eine Gruppe der Formel VII sein kann, $R_{19}$ Wasserstoff, $C_1$-$C_{12}$ Alkyl, $C_3$-$C_{12}$ Cycloalkyl oder eine Gruppe der Formel IX

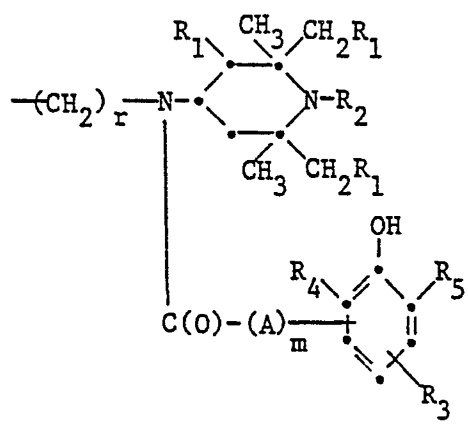

(IX)

ist oder $R_{18}$ und $R_{19}$ zusammen mit dem Stickstoffatom an dem sie gebunden sind einen 5- oder 6-gliedrigen Heterocyclus bilden, oder $R_{16}$ eine Gruppe der Formel IXa

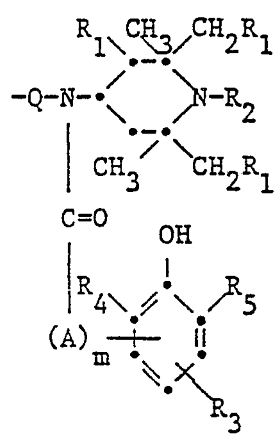

(IXa)

ist, worin Q die oben für B angegebene Bedeutung hat,

n und m unabhängig voneinander Null oder 1 bedeuten,

A Methylen oder eine Gruppe $—CH_2—CH(R_{20})—$ oder $—CH_2—C(E)(G)—$ ist, worin $R_{20}$ Wasserstoff, Methyl, Aethyl, Phenoxymethyl oder Phenyl ist, E Cyano oder eine Gruppe $—C(O)OR_{21}$, $—C(O)R_{22}$, $—SO_2R_{22}$, $—P(O)—(OR_{23})_2$, $—C(O)NR_{24}R_{25}$, —CHO ist, worin $R_{21}$ $C_1$-$C_4$ Alkyl, $R_{22}$ $C_1$-$C_{12}$ Alkyl, $C_7$-$C_{14}$ Alkaryl, Phenyl, $R_{23}$ $C_1$-$C_{18}$ Alkyl, Phenyl oder Allyl, $R_{24}$ und $R_{25}$ unabhängig voneinander Wasserstoff, $C_1$-$C_{18}$ Alkyl, Phenyl bedeuten und G Wasserstoff, $C_1$-$C_{18}$ Alkyl, $C_3$-$C_{12}$ Alkenyl, $C_3$-$C_4$ Alkinyl, $C_5$-$C_{12}$ Cycloalkyl, $C_6$-$C_{18}$ Alkylcycloalkyl, $C_6$-$C_{14}$ Cycloalkylalkyl, $C_7$-$C_{14}$ Aralkyl, $C_7$-$C_{19}$ Alkylaralkyl, Phenyl, durch Phenoxy, $C_7$-$C_{10}$ Alkylphenoxy, Benzyloxy, Cyclohexyloxy, Cyano, $—COOR_{26}$, $—OCOR_{27}$ oder $—P(O)(OR_{28})_2$ substituiertes $C_1$-$C_{18}$ Alkyl ist, wobei $R_{26}$ $C_1$-$C_{18}$ Alkyl, $C_3$-$C_{12}$ Cycloalkyl oder eine Gruppe der Formel VII, $R_{27}$ $C_1$-$C_{18}$ Alkyl, $C_3$-$C_{12}$ Cycloalkyl, Phenyl, $C_7$-$C_{14}$ Aralkyl oder eine Gruppe der Formel III, $R_{28}$ $C_1$-$C_{18}$ Alkyl, Allyl oder Phenyl bedeuten, oder G durch —O—, —S—, —SO—, $—SO_2—$ unterbrochenes $C_2$-$C_{18}$ Alkyl,

eine Gruppe der Formel VII oder eine Gruppe der Formel

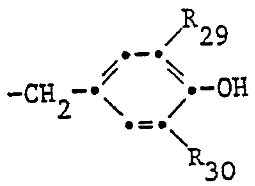

ist, worin $R_{29}$ und $R_{30}$ unabhängig voneinander $C_1$-$C_4$ Alkyl bedeuten und $R_{30}$ zusätzlich auch Wasserstoff sein kann, oder, wenn E eine Gruppe $—C(O)R_{22}$ ist, G und $R_{22}$ zusammen Trimethylen oder Tetramethylen bedeuten.

Bedeuten etwaige Substituenten $C_1$-$C_{12}$ Alkyl, so kann es sich um verzweigtes oder unverzweigtes Alkyl, wie z. B. Methyl, Aethyl, n-Propyl, Isopropyl, n-Butyl, sec.-Butyl, tert.-Butyl, n-Pentyl, n-Hexyl, n-Octyl, 1,1,3,3-Tetramethylbutyl, n-Nonyl, Decyl oder Dodecyl handeln. Bevorzugt ist $C_1$-$C_4$ Alkyl. $R_{16}$ und $R_{23}$ bedeuten als $C_1$-$C_{18}$ Alkyl zusätzlich z. B. Tridecyl, Tetradecyl, Hexadecyl oder Octadecyl.

Bedeuten etwaige Substituenten $C_1$-$C_4$ Alkyl so stellen sie Methyl, Aethyl, n-Propyl, Isopropyl, n-Butyl, sec.-Butyl oder tert.-Butyl dar.

Bedeuten etwaige Substituenten $C_3$-$C_{12}$ Alkenyl, so handelt es sich z. B. um Allyl, Methallyl, 2-Butenyl, 2-Hexenyl, 2-Octenyl, 4-Octenyl, 2-Decenyl oder 2-Dodecenyl, Bevorzugt ist Allyl. Substituenten die $C_2$-$C_{12}$ Alkenyl bedeuten, können zusätzlich auch Vinyl sein. In diesem Falle sind Vinyl und Allyl bevorzugt.

$R_2$, G und L bedeuten als $C_3$-$C_4$ Alkinyl z. B. Propargyl, n-But-1-inyl oder n-But-2-inyl. Bevorzugt ist Propargyl.

Als $C_3$-$C_{11}$ Alkoxyalkyl bedeuten $R_2$ und $R_{16}$ beispielsweise Aethoxymethyl, 2-Methoxyäthyl, 2-Aethoxyäthyl, 2-n-Butoxyäthyl, 2-n-Butoxypropyl, 2-n-Octoxyäthyl, 3-n-Octoxypropyl oder 6-n-Butoxyhexyl. $R_2$ kann als $C_2$ Alkoxyalkyl zusätzlich noch Methoxymethyl sein.

$X_1$ und $X_2$ bedeuten als Halogen z. B. Brom, Jod und insbesondere Chlor.

Bedeuten etwaige Substituenten $C_3$-$C_{12}$ Cycloalkyl, so handelt es sich z. B. um Cyclopropyl, Cyclopentyl, Cyclohexyl, Cycloheptyl, α-Methylcyclohexyl, Cyclooctyl, Cyclononyl, Cyclodecyl oder Cyclododecyl. Bevorzugt ist Cyclohexyl.

Bedeuten etwaige Substituenten $C_7$-$C_{14}$ Alkaryl, so handelt es sich z. B. um durch $C_1$-$C_4$ Alkyl substituiertes Phenyl, wie p-Tolyl, 2,4-Dimethylphenyl, 2,6-Dimethylphenyl, 2,4-Diäthylphenyl, 2,6-Diäthylphenyl, 4-tert.-Butylphenyl, 2,4-Di-tert.-butylphenyl oder 2,6-Di-tert.-butylphenyl. Bevorzugt sind 2,4-Di-tert.-butylphenyl und 2,4-Dimethylphenyl.

Bilden $R_7$ und $R_8$ bzw. $R_{18}$ und $R_{19}$ zusammen mit dem Stickstoffatom an dem sie gebunden sind einen 5- oder 6-gliedrigen Heterocyclus, so handelt es sich zum Beispiel um Pyrrolidin-, Oxazolidin-, Piperidin- oder Morpholinreste.

Bedeuten etwaige Substituenten $C_7$-$C_{14}$ Aralkyl, so stellen sie z. B. Benzyl, p-Methylbenzyl, p-tert.-Butylbenzyl, 1-Phenyläthyl, α,α-Dimethylbenzyl oder 2-Phenyläthyl dar.

Bedeuten B, $B_1$ oder Q eine Gruppe $—C_rH_{2r}—$, worin r eine Zahl zwischen 1 und 12 ist, so bedeutet r bevorzugt eine Zahl zwischen 2 und 8. Beispiele sind Methylen, Aethylen, Trimethylen, Tetramethylen, Hexamethylen, Octamethylen, Nonamethylen, 2,2,4-Trimethylhexamethylen, Decamethylen, Dodecamethylen.

Sind B oder Q $C_4$-$C_8$ Alkenylen, so handelt es sich z. B. um 2-Butenylen-1,4.

Sind B oder Q $C_4$-$C_8$ Alkinylen, so handelt es sich z. B. um 2-Butinylen-1,4.

B und Q bedeuten als $C_5$-$C_{12}$ Cycloalkylen z. B. Cyclopentylen, Cyclohexylen, Cyclooctylen, Cyclodecylen oder Cyclododecylen, Bevorzugt ist Cyclohexylen.

In der Bedeutung als durch Phenoxy, Benzyloxy, Cyclohexyloxy oder Cyano substituiertes $C_1$-$C_{12}$ Alkyl kann G beispielsweise einer der folgenden Reste sein : 2-Phenoxyäthyl, 2-Benzyloxyäthyl, Cyclohexyloxymethyl, 2-Cyanoäthyl, Cyanomethyl oder 3-Cyanopropyl.

G bedeutet als $C_6$-$C_{18}$ Alkylcycloalkyl z. B. Methylcyclohexyl, Aethylcyclohexyl, Butylcyclohexyl, tert.-Butylcyclohexyl, Dodecylcyclohexyl, Aethylcyclopentyl oder Butylcyclopentyl und als $C_6$-$C_{14}$ Cycloalkylalkyl beispielsweise Cyclohexylmethyl, Cyclohexyläthyl, Cyclohexylbutyl, Cyclohexylhexyl, Cyclohexyloctyl, Cyclopentylpropyl oder Cyclopentylhexyl.

Als durch $C_7$-$C_{10}$ Alkylphenoxy substituiertes $C_1$-$C_{18}$ Alkyl kann G beispielsweise p-Methylphenoxymethyl, p-Methylphenoxyäthyl, p-Methylphenoxypropyl, p-tert.-Butylphenoxymethyl, p-tert.-Butylphenoxyäthyl, 2,4-Dimethylphenoxymethyl, 2,4-Dimethylphenoxyäthyl, 2,4-Di-tert.-butylphenoxyäthyl, 2,6-Di-tert.-butylphenoxymethyl oder 2,4,6-Trimethylphenoxyäthyl sein.

In der Bedeutung als durch —O—, —S—, —SO— oder —$SO_2$— unterbrochenes $C_2$-$C_{12}$ Alkyl ist G z. B. einer der folgenden Reste : Methoxymethyl, 2-Butoxyäthyl, 2-Octyloxyäthyl, Isopropoxymethyl, 3-Butylthiopropyl, 2-Decylthioäthyl, 2-(Isohexylsulfinyl)-äthyl, 2-(Butylsulfonyl)-äthyl oder 2-(Aethylsulfonyl)-propyl.

Bedeutet $R_2$ eine Gruppe der Formel V oder der Formel VI und bedeutet oder enthält $R_{16}$ eine Gruppe der Formel VII oder der Formel IX, so können in der Verbindung der Formel I insgesamt nicht mehr als zwei Gruppen der Formel VII enthalten sein.

Bevorzugt werden solche farbphotographische Aufzeichnungsmaterialien, die als Lichtschutzmittel mindestens eine Verbindung der Formel X

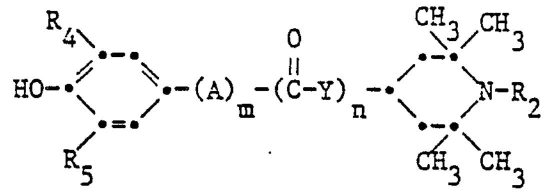

(X)

enthalten, worin $R_4$ und $R_5$ unabhängig voneinander $C_1$-$C_4$ Alkyl sind und $R_4$ zusätzlich Wasserstoff oder eine Gruppe der Formel Xa

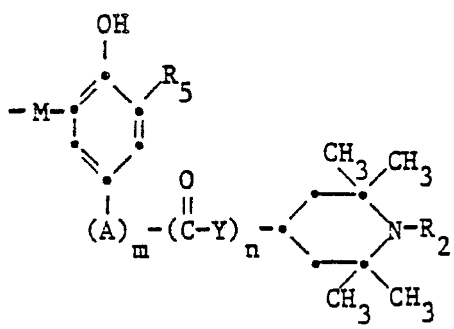

(Xa)

bedeutet, worin M eine direkte Bindung, —$CH(D_2)$— oder —S— und $D_2$ Wasserstoff oder $C_1$-$C_8$ Alkyl bedeuten,

A Methylen, Aethylen oder eine Gruppe —$CH_2$—C(E)(G)— ist, worin E Cyano, —$COCH_3$ und $COOCH_3$ und G Wasserstoff, $C_1$-$C_{18}$ Alkyl, Allyl, Cyclohexyl, Benzyl oder eine der beiden Gruppen der Formeln

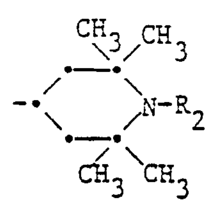

und

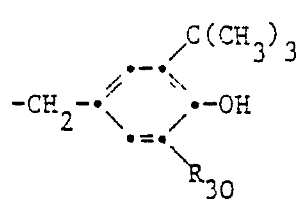

bedeuten, worin $R_{30}$ Methyl oder tert.-Butyl ist, oder E und G zusammen eine Gruppe —CO—$(CH_2)_4$— bilden,

n und m die oben angegebene Bedeutung haben,

$R_2$ Hydroxy, $C_1$-$C_4$ Alkyl, Allyl, Methallyl, Propargyl, Benzyl, 2-Hydroxyäthyl, $C_2$-$C_7$ Alkoxyalkyl, eine Gruppe der Formel —$(CH_2)_p$—$CH(R_6)$—$X_1$ oder —$CH(R_6)$—$X_2$ ist, worin p die Zahlen 1, 2 oder 3 bedeutet, $X_1$ Halogen, Cyano, —$OR_7$, —$OC(O)R_7$, —$OC(O)N(R_7)(R_8)$, —$C(O)R_7$, —$C(O)N(R_7)(R_8)$, $X_2$ Halogen, Cyano, 1,2-Epoxyäthyl, —$C(O)OR_7$, —$C(O)N(R_7)(R_8)$ sind und $R_6$ Wasserstoff, Methyl oder Phenyl ist, wobei $R_7$ Wasserstoff, $C_1$-$C_8$ Alkyl, Vinyl, Allyl, Methallyl, $C_5$-$C_8$ Cycloalkyl, Phenyl, $C_7$-$C_{10}$ Aralkyl und $R_8$ Wasserstoff und Methyl bedeuten, oder $R_2$ eine Gruppe der Formel II bedeutet, worin L

Wasserstoff, $C_1$-$C_4$ Alkyl, Vinyl, Allyl, Cyclohexyl, Phenyl, Benzyl, Chlormethyl oder eine Gruppe —$OR_9$ ist, worin $R_9$ $C_1$-$C_8$ Alkyl, Cyclohexyl, Vinyl, Allyl oder Methallyl bedeutet oder L eine Gruppe der Formel III ist, worin m, A, $R_4$ und $R_5$ die in dieser Bevorzugung angegebene Bedeutung haben und $R_3$ Wasserstoff ist, oder $R_2$ eine Gruppe der Formel IV ist, worin $R_{10}$ und $R_{11}$ unabhängig voneinander $C_1$-$C_{12}$ Alkyl, Cyclohexyl und Phenyl bedeuten und $R_{11}$ zusätzlich auch Wasserstoff sein kann, oder $R_2$ eine Gruppe der Formel XI

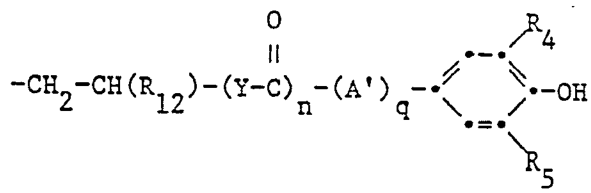

(XI)

ist, worin n, $R_4$, $R_5$ und Y die für diese Bevorzugung angegebene Bedeutung haben,

A' Methylen oder eine Gruppe —$CH_2$—$CH(R_{13})$— ist, q Null oder 1 bedeutet und $R_{12}$ und $R_{13}$ unabhängig voneinander Wasserstoff oder Methyl sind, oder $R_2$ eine Gruppe der Formel XII

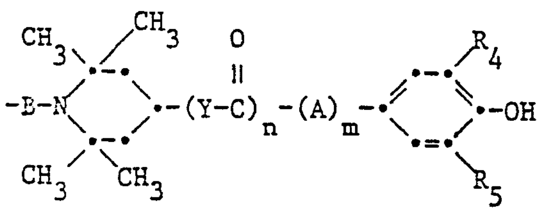

(XII)

ist, worin A, $R_4$, $R_5$, Y, n und m die für diese Bevorzugung angegebene Bedeutung haben und B eine Gruppe —(—$CH_2$—)—$_r$ oder —CONH—(—$CH_2$—)$_r$—NHCO—, worin r die Zahlen 2 bis 8 bedeutet, oder $C_4$-$C_8$ Alkenylen, Xylylen oder Bitolylen ist, oder $R_2$ eine der Gruppen —$SO_2R_{14}$ oder —$OR_{15}$ bedeutet, worin $R_{14}$ $C_1$-$C_4$ Alkyl, p-Tolyl oder Phenyl ist und $R_{15}$ $C_1$-$C_4$ Alkyl, Benzyl oder eine Gruppe der Formel L'—CO— bedeutet, worin L' die in dieser Bevorzugung bereits für L angegebene Bedeutung hat,

Y —O— oder —$N(R_{16})$— ist, worin $R_{16}$ Wasserstoff, $C_1$-$C_{12}$ Alkyl, Cyclohexyl, $C_3$-$C_7$ Alkoxyalkyl oder eine Gruppe der Formel XIII

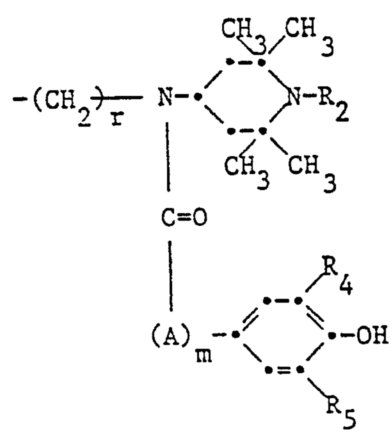

(XIII)

ist, worin r, m, A, $R_2$, $R_4$ und $R_5$ die in dieser Bevorzugung bereits angegebene Bedeutung haben.

Besonders bevorzugt werden farbphotographische Aufzeichnungsmaterialien, die als Lichtschutzmittel mindestens eine Verbindung der Formel XIV

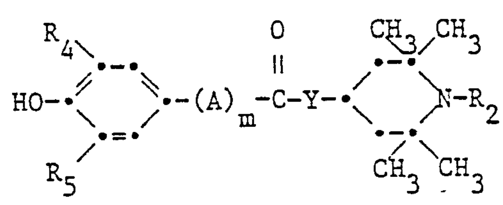

(XIV)

enthalten, worin m Null oder 1 ist

A Methylen oder Aethylen bedeutet

$R_4$ und $R_5$ unabhängig voneinander Methyl oder tert.-Butyl sind
Y —O— oder —N($R_{16}$)—, worin $R_{16}$ Wasserstoff oder $C_1$-$C_8$ Alkyl ist, und
$R_2$ Hydroxy, Methyl, Allyl, Benzyl, 2-Hydroxyäthyl, Acetyl, Acryloyl, Methoxy, Acetoxy oder eine Gruppe der Formel XV

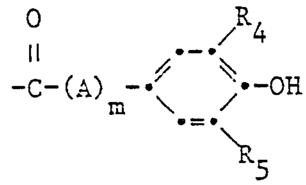

(XV)

worin m, A, $R_4$ und $R_5$ die für diese Bevorzugung angegebene Bedeutung haben, oder eine Gruppe der Formel IV, worin $R_{10}$ $C_1$-$C_8$ Alkyl, Cyclohexyl oder Phenyl und $R_{11}$ Wasserstoff, $C_1$-$C_8$ Alkyl oder Cyclohexyl sind.

Ebenfalls besonders bevorzugt werden farbphotographische Aufzeichnungsmaterialien, die als Lichtschutzmittel mindestens eine Verbindung der Formel XIV enthalten, worin $R_2$ eine Gruppe der Formel

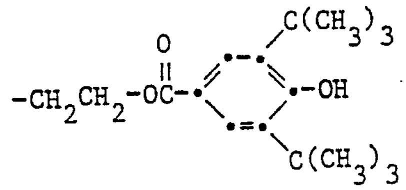

bedeutet und m, A, $R_4$, $R_5$ und Y die in der letzten Bevorzugung angegebene Bedeutung haben, sowie farbphotographische Aufzeichnungsmaterialien, die als Lichtschutzmittel mindestens eine Verbindung der Formel XIV enthalten, worin A eine Gruppe —$CH_2$—C(E)(G)— ist, in welcher E Cyano, —$COCH_3$ und —$COOCH_3$ und G Wasserstoff oder eine der beiden Gruppen der Formeln

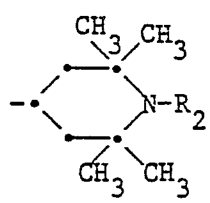

und

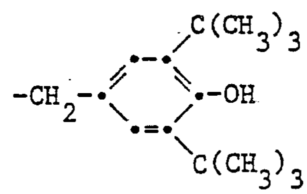

bedeuten oder E und G zusammen eine Gruppe —CO—$(CH_2)_4$— bilden, $R_2$ —CHO, Acetyl oder Acryloyl bedeutet und m, $R_4$, $R_5$ und Y die in der letzten Bevorzugung angegebene Bedeutung haben.

Von besonderem Interesse sind farbphotographische Aufzeichnungsmaterialien, die als Lichtschutzmittel mindestens eine Verbindung der Formel I enthalten, worin Y —N($R_{16}$)— und n die Zahl 1 bedeuten. Diese Verbindungen sind neu und als solche auch Gegenstand der vorliegenden Erfindung.

Sie können nach an sich bekannten Methoden, durch Amidierung von bekannten 4-Aminopiperidinverbindungen, etwa nach der Gleichung

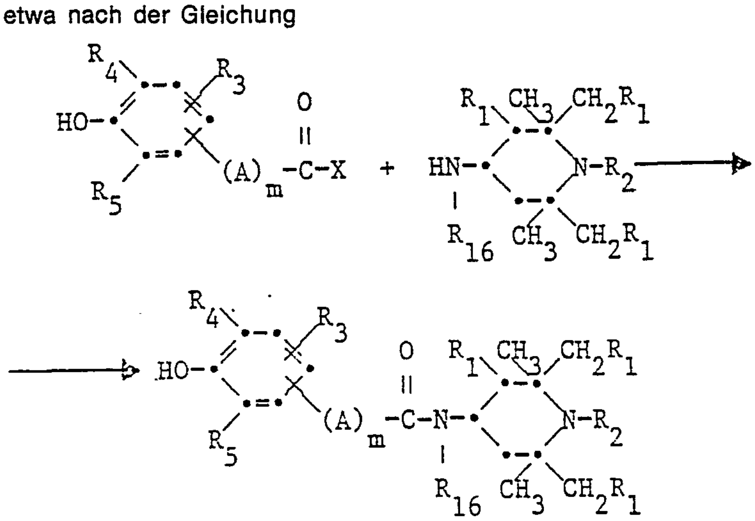

hergestellt werden, wobei X Chlor oder —OR ist, worin R Methyl oder Aethyl bedeutet und $R_1$, $R_2$, $R_3$, $R_4$, $R_5$, $R_{16}$, A und m die oben angegebene Bedeutung haben.

Ebenfalls interessant sind farbphotographische Aufzeichnungsmaterialien, die als Lichtschutzmittel mindestens eine Verbindung der Formel I enthalten, worin A eine Gruppe $—CH_2—C(E)(G)—$, m und n die Zahl 1 und $R_2$ —OH, $—SO_2R_{14}$, $—OR_{15}$ oder eine Gruppe der Formeln V oder VI bedeuten. Auch diese Verbindungen sind neu. Sie können ebenso nach an sich bekannten Methoden, und gemäss der Gleichung

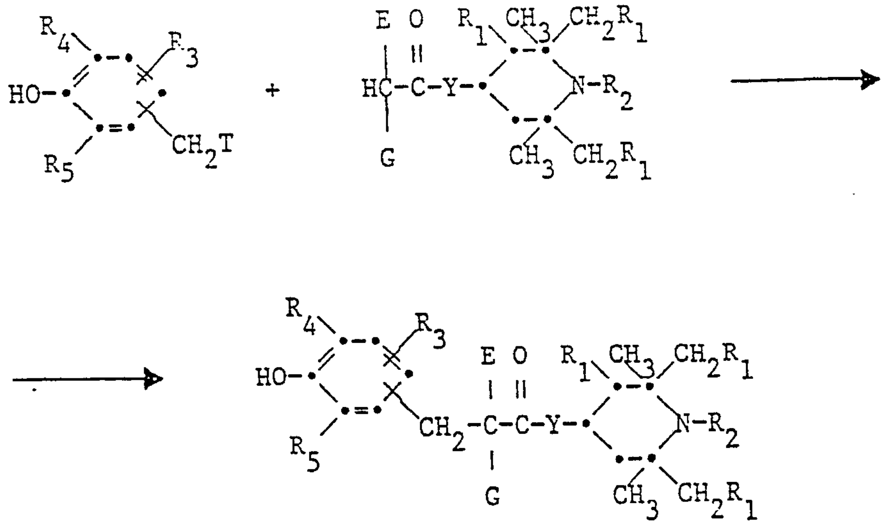

hergestellt werden, wobei T eine Gruppe $—N(R)_2$, $—SC(S)N(R)_2$ oder —OR ist, worin R Methyl oder Aethyl bedeutet, $R_2$ —OH, $—SO_2R_{14}$, $—OR_{15}$ oder eine Gruppe der Formeln V oder VI ist und $R_1$, $R_3$, $R_4$, $R_5$, E, G und Y die oben angegebene Bedeutung haben.

In einer Variante ist es in beiden Fällen möglich die genannten Verbindungen durch bekannte Reaktionen am Ringstickstoff der Verbindungen der Formel

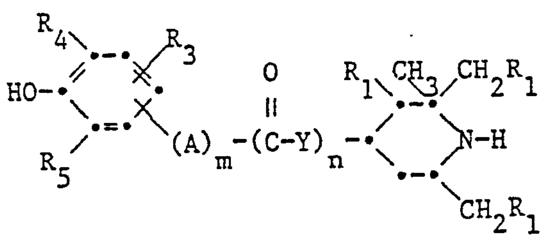

z. B. durch Alkylierung, Acylierung oder Addition herzustellen.

Die Ausgangssubstanzen sowie die anderen Verbindungen der Formel I sind bekannt. Sollten einzelne von ihnen noch neu sein, so können sie in Analogie zu an sich bekannten Methoden hergestellt werden.

Typische Vertreter von Verbindungen der Formel I sind in den nachstehenden Tabellen I bis V aufgeführt.

(Siehe Tabellen Seite 10 ff.)

Die Verbindungen der Formel I sind in Wasser kaum löslich, und sie werden deshalb in einem niedrigsiedenden organischen Lösungsmittel, wie Methylacetat, Aethylacetat, Tetrachlorkohlenstoff, Chloroform, Methanol, Aethanol, n-Butanol, Dioxan, Aceton oder Benzol, einem hochsiedenden organischen Lösungsmittel, wie Tricresylphosphat, Dimethylformamid, Dimethylsulfoxid, Di-n-butylphthalat oder Aethyl-N-diphenylcarbamat, oder einem Lösungsmittelgemisch aus den oben erwähnten niedrigsiedenden und hochsiedenden organischen Lösungsmitteln gelöst, die erhaltene Lösung wird zu einer Schutzkolloidlösung, wie insbesondere einer wässrigen Gelatinelösung gegeben und mittels einer Kolloidmühle, eines Homogenisators oder durch Anwendung von Ultraschall dispergiert.

Die so erhaltenen Dispersionen werden dann zur Herstellung der Schichten von farbphotographischen Aufzeichnungsmaterialien verwendet. Diese Schichten können z. B. Zwischen- oder Schutzschich-

Tabelle I

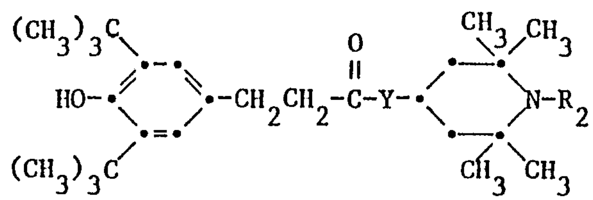

| Lichtschutz-mittel Nr. | Y | $R_2$ |
|---|---|---|
| 1 | -NH- | $-CH_3$ |
| 2 | -NH- | $-CO-CH_3$ |
| 3 | $-N(C_4H_9)-$ | $-CO-CH_3$ |
| 4 | -NH- | $-CO-CH=CH_2$ |
| 5 | $-N(C_4H_9)-$ | $-CO-CH=CH_2$ |
| 6 | -O- | $-CH_3$ |
| 7 | -O- | $-CH_2-$ 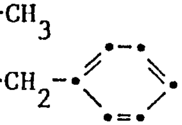 |
| 8 | -O- | $-CO-CH_3$ |
| 9 | -O- | $-CO-CH=CH_2$ |
| 10 | -O- | $-CH_2-CH=CH_2$ |

Tabelle I (Fortsetzung)

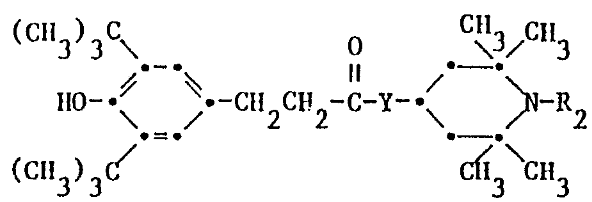

| Lichtschutz-mittel Nr. | Y | $R_2$ |
|---|---|---|
| 11 | -O- | $-CH_2CN$ |
| 12 | $>N-C_8H_{17}$ | $-CO-CH_3$ |
| 13 | $>N-C_8H_{17}$ | $-CO-CH=CH_2$ |
| 14 | -O- | 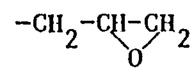 |
| 15 | 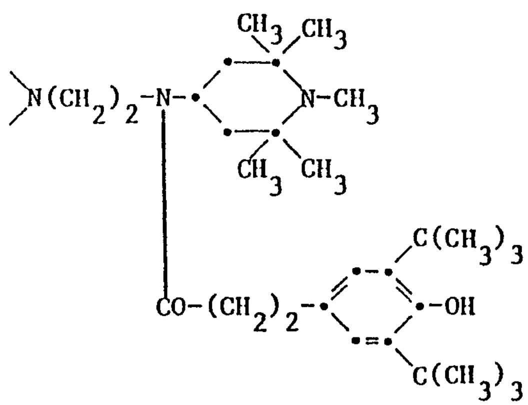 | $-CH_3$ |

Tabelle I (Fortsetzung)

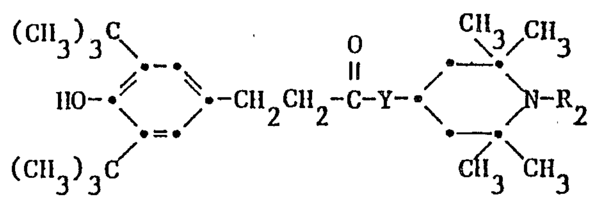

| Lichtschutz-mittel Nr. | Y | $R_2$ |
|---|---|---|
| 16 | -O- | $-CH_3-COOCH_3$ |
| 17 | -O- | 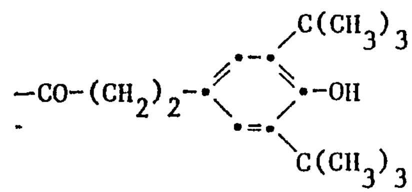 |
| 18 | -O- | $-CH_2CH_2OH$ |
| 19 | -O- | $-CH_2-C{\equiv}CH$ |
| 20 | -O- | $-CON(C_2H_5)_2$ |
| 21 | -O- | $-OCH_3$ |

Tabelle I (Fortsetzung)

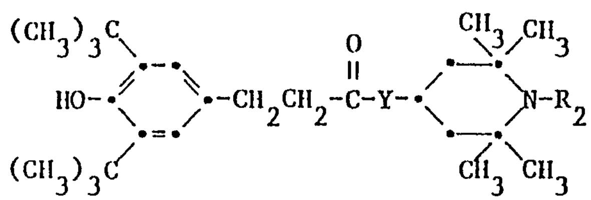

| Lichtschutz-mittel Nr. | Y | $R_2$ |
|---|---|---|
| 22 | -O- | 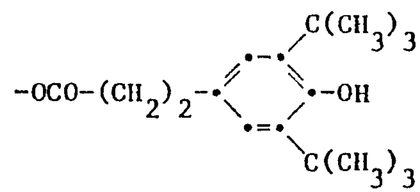 |
| 23 | -O- | 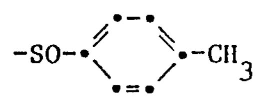 |
| 24 | -O- | 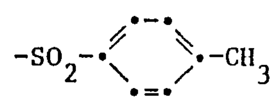 |

Tabelle I (Fortsetzung)

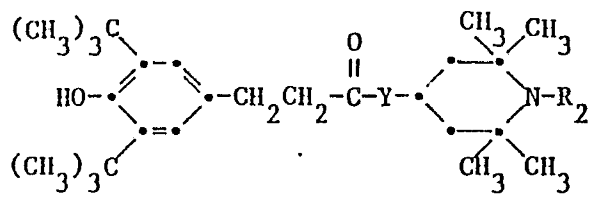

| Lichtschutzmittel Nr. | Y | $R_2$ |
|---|---|---|
| 25 | 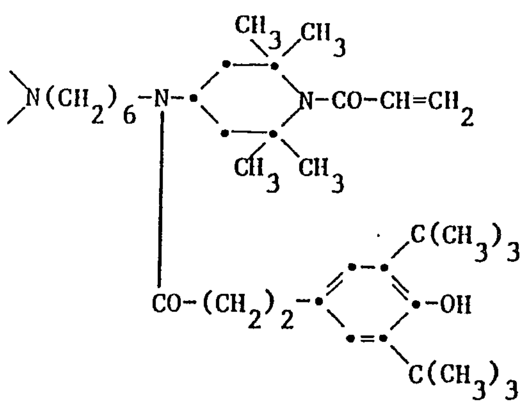 | $-COCH=CH_2$ |
| 26 | 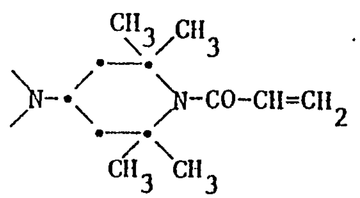 | $-CO-CH=CH_2$ |

Tabelle I (Fortsetzung)

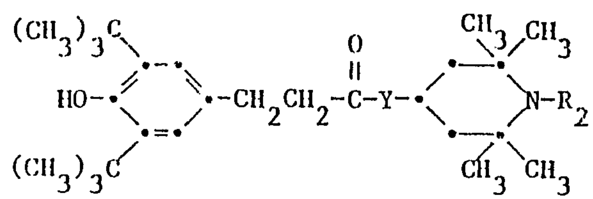

| Lichtschutz-mittel Nr. | Y | $R_2$ |
|---|---|---|
| 27 | -O- | 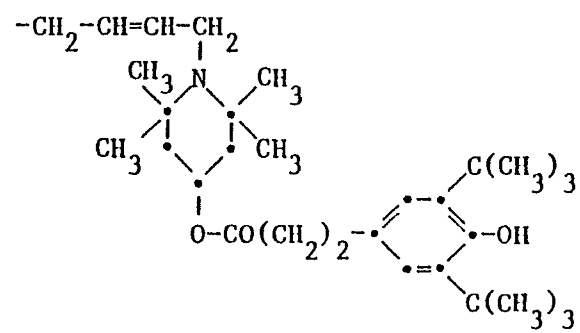 |
| 28 | -O- | 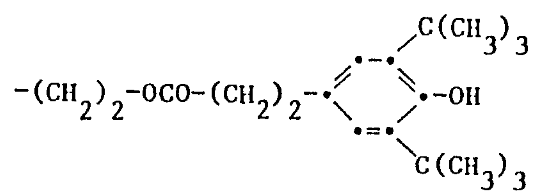 |
| 29 | $>N-C_4H_9$ | $-CH_3$ |

Tabelle II

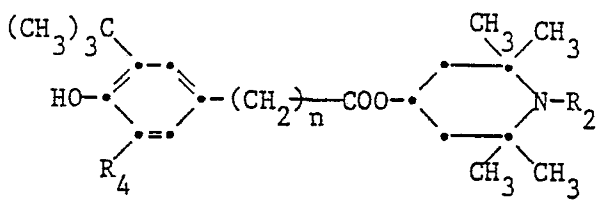

| Lichtschutzmittel Nr. | $R_4$ | n | $R_2$ |
|---|---|---|---|
| 30 | -H | 2 | $-CO-CH=CH_2$ |
| 31 | -H | 2 | $-CH_3$ |
| 32 | $-CH_3$ | 2 | $-CH_3$ |
| 33 | $-CH_3$ | 2 | $-CO-CH=CH_2$ |
| 34 | $-CH_3$ | 2 | $-CO-CH_3$ |
| 35 | -H | 2 | $-CO-CH_3$ |
| 36 | $-C(CH_3)_3$ | 0 | 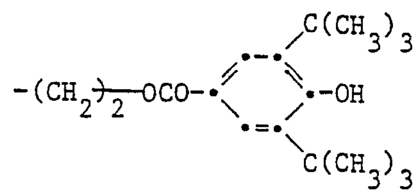 |
| 37 | $-C(CH_3)_3$ | 0 | $-CO-CH=CH_2$ |
| 38 | $-C(CH_3)_3$ | 1 | $-CO-CH=CH_2$ |

Tabelle III

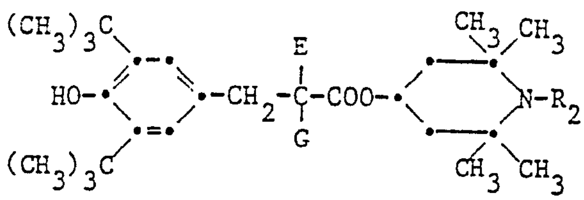

| Lichtschutz-mittel Nr. | E | G | $R_2$ |
|---|---|---|---|
| 39 | $-CN$ | $-H$ | $-CHO$ |
| 40 | $-CO-CH_3$ | 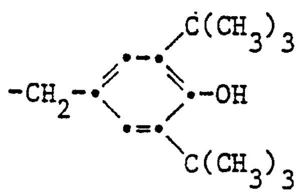 | $-CO-CH_3$ |
| 41 | $-COOCH_3$ | 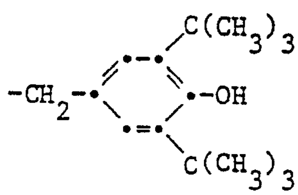 | $-CO-CH_3$ |
| 42 | $-CN$ | 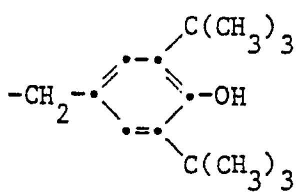 | $-CO-CH=CH_2$ |
| 43 | $-CN$ | 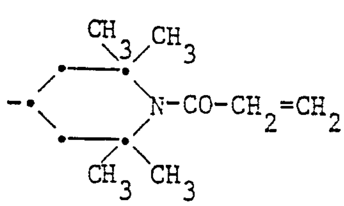 | $-CO-CH=CH_2$ |
| 44 | 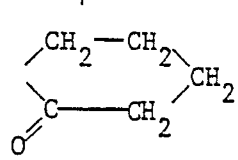 | | $-CO-CH=CH_2$ |

Tabelle IV

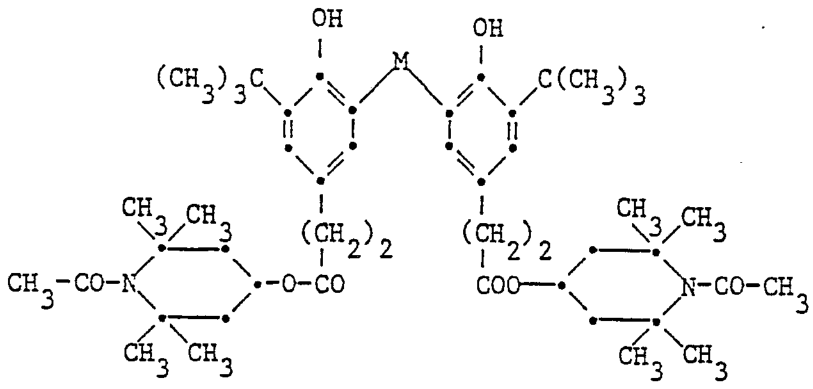

| Lichtschutzmittel Nr. | M |
|---|---|
| 45 | -S- |
| 46 | $-CH_2-$ |
| 47 | direkte Bindung |

Tabelle V

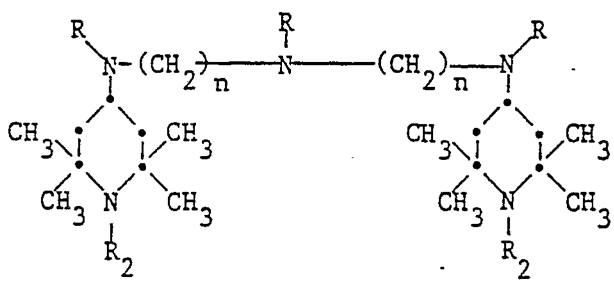

| Lichtschutz-mittel Nr. | n | R | $R_2$ |
|---|---|---|---|
| 48 | 2 | 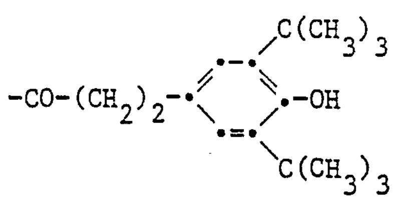 | $-CO-CH_3$ |
| 49 | 3 | 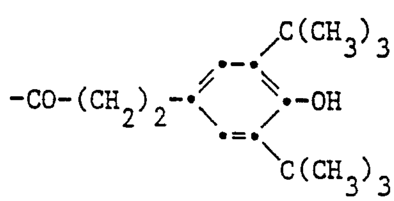 | $-CO-CH=CH_2$ |

ten, insbesondere jedoch lichtempfindliche (z. B. blau-, grün- und rotempfindliche Silberhalogenidemulsionsschichten sein, in denen bei der Entwicklung des belichteten Aufzeichnungsmaterials aus den entsprechenden Farbkupplern, die Blaugrün (Cyan)-, Purpur (Magenta)- und Gelbfarbstoffe gebildet werden.

Gegebenenfalls kann das Lichtschutzmittel auch in den Behandlungsbädern appliziert werden, die nach der Farbentwicklung verwendet werden, z. B. in Fixier- und/oder Waschbädern, wobei jedoch eine gewisse Löslichkeit der Verbindungen der Formel I in Alkoholen, (Methanol/Aethanol) wässrigem Alkali und/oder Wasser erforderlich ist. Wenn die Diffusionstransfermethode angewandt wird, kann das Lichtschutzmittel nicht nur in die üblichen photographischen Emulsionsschichten, sondern auch in eine Empfangsschicht eingearbeitet werden.

Beliebige Blaugrün-, Purpur- und Gelb-Kuppler, die zur Bildung der genannten Farbstoffe und damit der Farbbilder verwendet werden, können eingesetzt werden. Sie können z. B. in alkalischer Lösung oder in einem hochsiedenden organischen Lösungsmittel gelöst werden, wobei dann diese Lösungen in einer wässrigen Gelatinelösung dispergiert und in eine photographische Silberhalogenidemulsion eingearbeitet werden. Sie können auch farbphotographischen Entwicklern beigefügt werden.

Im photographischen Aufzeichnungsmaterial gemäss vorliegender Erfindung können die Lichtschutzmittel gemäss Formel I ausser mit den Farbkupplern zusätzlich auch mit Ultraviolettabsorbern in der gleichen Schicht kombiniert werden.

Die Silberhalogenidemulsionen enthalten als Bindemittel vorzugsweise Gelatine, gegebenenfalls im Gemisch mit anderen hochmolekularen natürlichen oder synthetischen Verbindungen.

Die Silberhalogenidemulsionen können beispielsweise Silberbromid-, Silberchlorid- oder Silberjodidemulsionen oder auch solche Emulsionen sein, die ein Gemisch von Silberhalogeniden enthalten, wie z. B. Silberbromid-jodid- oder Silberchlorid-bromidemulsionen.

Die Emulsionen können chemisch sensibilisiert werden, sie können ferner übliche organische Stabilisatoren und Antischleiermittel sowie auch übliche Weichmacher, wie z. B. Glycerin, enthalten. Die Emulsionen können ferner mit den für Gelatine üblichen Härtungsmitteln gehärtet werden. Ferner können die Emulsionen übliche Giesshilsmittel enthalten. Die Emulsionen können auf übliche Schichtträger für photographisches Aufzeichnungsmaterial aufgebracht werden.

Zur Entwicklung des farbenphotographischen Aufzeichnungsmaterials können die üblichen Entwicklerbäder eingesetzt werden. Diese enthalten in der Regel eine Entwicklersubstanz des p-Phenylendiamin-Typs, einen Entwicklungsverzögerer, wie Kaliumbromid, ein Antioxydationsmittel, wie Natriumsulfit, und eine Base, z. B. ein Alkalihydroxyd oder Alkalicarbonat. Ferner können die Entwicklungsbäder ein übliches Antischleiermittel und Komplexbildner enthalten.

Die erfindungsgemäss einzusetzenden Lichtschutzmittel eignen sich in gewissen Fällen auch zum Schutz farbphotographischer Schichten in denen die Farbstoffe direkt in die Emulsion eingelagert werden und das Bild durch selektive Bleichung erzeugt wird.

Die Menge des Lichtschutzmittels, gegebenenfalls in Kombination mit einem üblichen Ultraviolettabsorber, kann in weiten Grenzen schwanken und liegt etwa im Bereich von 1 bis 2 000 mg, vorzugsweise 1 bis 800 und insbesondere 400 mg pro $m^2$ der Schicht, in die es (sie) eingearbeitet wird (werden).

Falls das photographische Material ein UV-Licht absorbierendes Mittel enthält, so kann dieses in der gleichen oder in der benachbarten Schicht enthalten sein.

Der Ultraviolettabsorber kann mit dem Lichtschutzmittel zusammen in einer Schicht oder auch in einer benachbarten Schicht vorhanden sein. Das Gewichtsverhältnis zwischen einem üblichen Ultraviolettabsorber und dem Lichtschutzmittel der Formel I beträgt etwa (5-10) : 1, das molare Verhältnis etwa (10-20) : 1. Beispiele für Ultraviolett-Absorber sind Verbindungen vom Benzophenon- Acrylnitril-, Thiazolidon-, Benztriazol-, Oxazol-, Thiazol- und Imidazoltyp. Die mit dem erfindungsgemässen Aufzeichnungsmaterial durch Belichtung und Entwicklung erhaltenen Farbbilder zeigen eine sehr gute Lichtechtheit gegenüber sichtbarem und ultraviolettem Licht. Die Verbindungen der Formel I sind praktisch farblos, so dass es zu keiner Verfärbung der Bilder kommt ; ausserdem sind sie gut verträglich mit den üblichen, in den einzelnen Schichten vorhandenen photographischen Zusatzstoffen ; aufgrund ihrer guten Wirksamkeit kann man ihre Einsatzmenge herabsetzen und vermeidet so ihre Ausfällung oder ihr Auskristallisieren, wenn man sie als organische Lösung in die wässerigen Bindemittelemulsionen, die für die Herstellung photographischer Schichten verwendet werden, einarbeitet. Die einzelnen nach der Belichtung des photographischen Aufzeichnungsmaterials notwendigen Verarbeitungsschritte zur Herstellung der Farbbilder werden durch die Lichtschutzmittel nicht nachteilig beeinflusst. Ferner kann die bei blauempfindlichen Emulsionen häufig auftretende sogenannte Druckschleierbildung weitgehend zurückgedrängt werden. Diese kann z. B. auftreten, wenn auf photographische Materialien (Silberhalogenidemulsionsschichten, die sich auf einem Träger aus natürlichen oder synthetischen Materialien befinden) mechanische Beanspruchungen, z. B. Drehen, Biegen oder Reiben, während der Herstellung oder während der Behandlung vor der Entwicklung ausgeübt werden. (T.H. James, The Theory of the Photographic Process 4. Auflage, Macmillan, New York, N.Y. 1977, Seite 23 ff., S. 166 ff.)

Die nachfolgenden Beispiele erläutern die vorliegende Erfindung ohne sie einzuschränken.

## Herstellungsbeispiele

## Beispiel 1

4,1 g 2,2,6,6-Tetramethylpiperidinyl-4–β-(3,5-di-tert.-butyl-4-hydroxyphenyl)-propionamid, 2,8 g Methyljodid und 6,5 g wasserfreies Kaliumcarbonat werden vier Stunden in 100 ml Aceton unter Rückfluss erhitzt. Das Aceton wird abgedampft und der Rückstand in 150 ml Aether gelöst, mit Wasser gewaschen und getrocknet. Nach Abdampfen des Lösungsmittels erhält man ein gelbes Oel, das aus Acetonitril kristallisiert. Man erhält 2,6 g (60 %) 1,2,2,6,6-Pentamethylpiperidinyl-4-β-(3,5-di-tert.-butyl-4-hydroxyphenyl)-propionamid als farblose Kristalle mit Smp. 132-134 °C (Lichtschutzmittel 1).

## Beispiel 2

4,1 g 2,2,6,6-Tetramethylpiperidinyl-4-β-(3,5-di-tert.-butyl-4-hydroxyphenyl)-propionamid werden zwölf Stunden in 80 ml Essigsäureanhydrid bei 90 °C gerührt. Das Lösungsmittel wird unter vermindertem Druck abgedampft, der Rückstand in 50 ml Methanol gelöst, und in 150 ml 100 %ige Kochsalzlösung langsam getropft. Der Feststoff wird abgenutscht, bei 50 °C getrocknet und in 50 ml Aceton gelöst. Die Acetonlösung wird durch Filtration geklärt, mit 150 ml Aether verdünnt, auf 0 °C abgekühlt und der erhaltene Niederschlag abgenutscht. So erhält man 3,1 g (69 %) 1-Acetyl-2,2,6,6-tetramethyl-piperidinyl-4-β-(3,5-di-tert.-butyl-4-hydroxyphenyl)-propionamid als farblose Kristalle mit Smp. 130-132 °C (Lichtschutzmittel 2).

## Beispiel 3

4,7 g 2,2,6,6-Tetramethylpiperidinyl-4-β-(3,5-di-tert.-butyl-4-hydroxyphenyl)-N-butyl-propionamid werden zwölf Stunden in 80 ml Essigsäureanhydrid bei 90 °C gerührt. Das Lösungsmittel wird unter vermindertem Druck abgedampft und das orangefarbene Oel in 60 ml Petroläther zehn Minuten bei 50 °C erhitzt. Der Niederschlag wird abgenutscht, mit Petroläther gewaschen und bei 50 °C getrocknet, Man erhält 3,5 g (68 %) 1-Acetyl-2,2,6,6-tetramethylpiperidinyl-4-β-(3,5-di-tert.-butyl-4-hydroxyphenyl)-N-butylpropionamid als farbloses Pulver mit Smp. 133-135 °C (Lichtschutzmittel 3).

## Beispiel 4

4,1 g 2,2,6,6-Tetramethylpiperidinyl-4-β-(3,5-di-tert.-butyl-4-hydroxyphenyl)-propionamid werden in 150 ml Acetonitril gelöst. 1,5 g Triäthylamin werden zugegeben und die Lösung auf 60 °C gewärmt. 1,0 g Acrylsäurechlorid wird tropfenweise zugegeben und nach der Zugabe eine weitere Stunde gerührt. Das Reaktionsgemisch wird filtriert und das Filtrat abgedampft. Nach Reinigung durch Säulechromatographie erhält man 2,0 g 1-Acryloyl-2,2,6,6-tetramethylpiperidinyl-4-β-(3,5-di-tert.-butyl-4-hydroxyphenyl)-propionamid mit Smp. 149 bis 151 °C (Lichtschutzmittel 4).

## Beispiel 5

Verfährt man wie in Beispiel 4 beschrieben, mit der Ausnahme, dass man die entsprechende Menge 2,2,6,6-Tetramethylpiperidinyl-4-β-(3,5-di-tert.-butyl-4-hydroxyphenyl)-N-butylpropionamid einsetzt, so erhält man 1-Acryloyl-2,2,6,6-tetramethylpiperidinyl-4-β-(3,5-di-tert.-butyl-4-hydroxyphenyl)-N-butylpropionamid mit Smp. 148 bis 149 °C (Lichtschutzmittel 5).

## Beispiel 6

10,8 g Cyanessigsäure-2,2,6,6-tetramethyl-4-piperidinylester und 35 g 2,6-Di-tert-butyl-4-hydroxybenzyl-N,N-di-äthyl-di-thiocarbamat werden in 150 ml Isopropanol vorgelegt und auf 50 °C erwärmt. Bei dieser Temperatur wird während 30 Minuten eine Lösung von 3,8 g Natrium-Hydroxid in 20 ml Wasser hinzugetropft. Die hellbraune Lösung wird bei 50-60 °C nachgerührt und anschliessend 1,5 Stunden unter Rückfluss gekocht. Die grüne Lösung wird auf Raumtemperatur abgekühlt, 200 ml Wasser zugesetzt und dann auf 5 °C abgekühlt. Das ausgefallene leicht gelbe Produkt wird abgenutscht, mit kaltem Isopropanol (wässrig) gewaschen und getrocknet. Smp. 174 °C. Dieses Produkt wird dann wie in Beispiel 4 beschrieben mit Acrylsäurechlorid zum α-(3,5-Di-tert-butyl-4-hydroxybenzyl)-α-cyano-β-(3,5-di-tert-butyl-4-hydroxyphenyl)-propionsäure-4-(-1-acryloyl-2,2,6,6-tetramethylpiperidinyl)-ester umgesetzt. Smp. 188-190 °C. (Lichtschutzmittel Nr. 42).

## Anwendungsbeispiele

### Beispiel 7a und b

0,087 g des Gelbkupplers der Formel

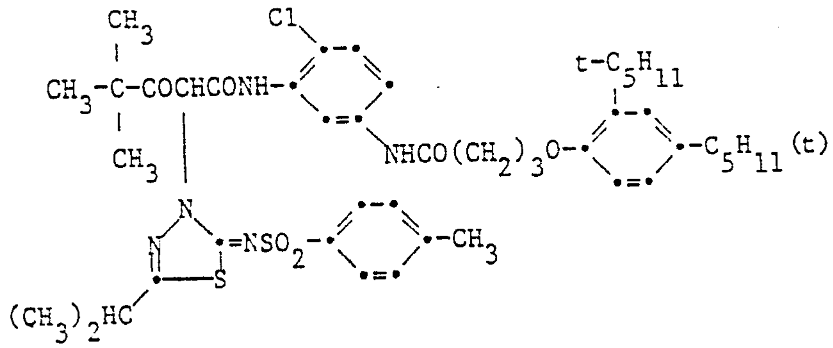

und 0,026 g eines der in den nachfolgenden Tabellen angegebenen Lichtschutzmittels werden in 2,0 ml eines Gemisches von Trikresylphosphat/Aethylacetat (1,5 g in 100 ml) gelöst. Zu dieser Lösung gibt man 7,0 ml einer 6 %igen Gelatinelösung, 0,5 ml einer 8 %igen Lösung des Netzmittels der Formel

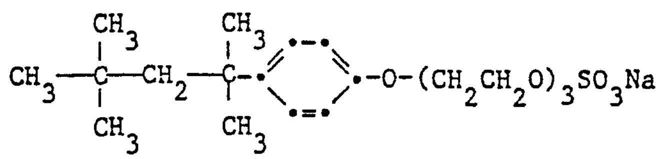

in Isopropanol/Wasser (3 : 4) und 0,5 ml Wasser und emulgiert mit Ultraschall bei einer Leistung von 100 Watt während 5 Minuten.

Zu 2,5 ml der so erhaltenen Emulsion gibt man 2,0 ml einer Silberbromid-Emulsion mit einem Silbergehalt von 6,0 g pro Liter, 0,7 ml einer 1 %igen wässerigen Lösung des Härters der Formel

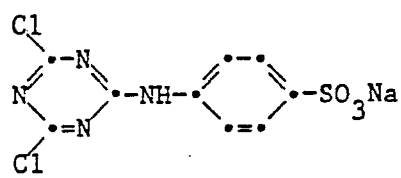

und 3,8 ml Wasser, stellt das Gemisch auf einen pH-Wert von 6,5 und vergiesst es auf ein auf eine Glasplatte aufgezogenes substriertes, kunststoffbeschichtetes, weisses Papier.

Nach dem Erstarren wird in einem Trockenschrank mit Umluft bei Raumtemperatur getrocknet.

Nach 3 Tagen werden auf 35 × 180 mm geschnittene Proben hinter einem Stufenkeil mit 3 000 Lux.s belichtet und anschliessend im Ektaprint 2®-Prozess der Firma Kodak verarbeitet.

a) Ein Teil der so erhaltenen Gelbkeile wird in einem Atlas Weather-O-meter mit einer 2 500 W-Xenonlampe hinter einem UV-Filter (Kodak 2C) mit 63 KJoule/cm² und ein zweiter Teil mit 168 KJoule/cm² bestrahlt (je eine weitere Vergleichsprobe enthält kein Lichtschutzmittel)

Die Tabelle drückt die prozentuale Abnahme der Dichte bei einer ursprünglichen Dichte von 1,0 aus :

Mit UV-Filter

| Lichtschutzmittel Nr. | Dichteverlust bei 440 nm in Prozent (D = 1,0), Lichtmenge | |
|---|---|---|
| | 63 KJ/cm² | 168 KJ/cm² |
| - | 13 | 28 |
| 9 | 5 | 13 |
| 27 | 5 | 15 |
| 28 | 5 | 12 |
| 29 | 5 | 13 |
| 36 | 5 | 13 |

b) Ein Teil der erhaltenen Gelbkeile wird wie unter a) beschrieben aber ohne UV-Filter mit 21 KJoule/$cm^2$ und ein weiterer Teil ebenfalls ohne UV-Filter mit 63 KJoule/$cm^2$ bestrahlt (je eine weitere Vergleichsprobe enthält kein Lichtschutzmittel).

Die Tabelle drückt die prozentuale Abnahme der Dichte bei einer ursprünglichen Dichte von 1,0 aus :

Ohne UV-Filter

| Lichtschutz-mittel Nr. | Dichteverlust bei 440 nm in Prozent (D = 1,0), Lichtmenge | |
|---|---|---|
| | 21 KJ/$cm^2$ | 63 KJ/$cm^2$ |
| - | 14 | 37 |
| 4 | 3 | 16 |
| 36 | 5 | 15 |

Beispiel 8

0,025 g des Cyankupplers der Formel

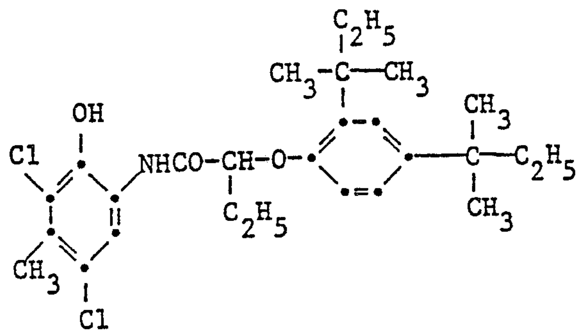

und 0,025 g eines Lichtschutzmittels der nachfolgenden Tabelle werden in 1 ml eines Gemisches von Trikresylphosphat-Aethylacetat (1,5 g in 100 ml) gelöst. Zu dieser Lösung gibt man 7,0 ml einer 6 %igen Gelatinelösung, 0,5 ml einer 8 %igen Lösung des Netzmittels der Formel

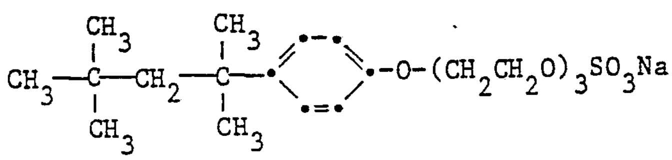

in Isopropanol/Wasser (3 : 4) und 0,5 ml Wasser und emulgiert mit Ultraschall bei einer Leistung von 100 Watt während 5 Minuten.

Zu 2,5 ml der so erhaltenen Emulsion gibt man 2,0 ml einer Silberbromid-Emulsion mit einem Silbergehalt von 6,0 g pro Liter, 0.7 ml einer 1 %igen wässerigen Lösung des Härters der Formel

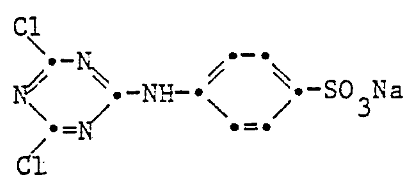

und 3,8 ml Wasser, stellt das Gemisch auf einen pH-Wert von 6,5 und vergiesst es auf ein auf eine

Glasplatte aufgezogenes substriertes, kunststoffbeschichtetes, weisses Papier.

Nach dem Erstarren wird in einem Trockenschrank mit Umluft bei Raumtemperatur getrocknet.

In Analogie zu dem in Beispiel 7a und b beschriebenen Verfahren werden Schichten hergestellt, belichtet und verarbeitet.

Die erhaltenen Keile werden in einem Klimaschrank bei 60 °C und 70 % rel. Feuchtigkeit gelagert. Die nachfolgende Tabelle drückt die prozentuale Abnahme der Cyandichte bei einer ursprünglichen Dichte von 1,0 im Rot aus (Messung mit einem Densitometer ® TR 924 Status A, der Firma Macbeth).

| Lichtschutz-mittel Nr. | Dichteverlust im Rot in Prozent (D = 1,0, 60°C/70% RF) | |
|---|---|---|
| | 14 Tage | 28 Tage |
| - | 32 | 46 |
| 36 | 15 | 25 |
| 40 | 16 | 26 |

**Patentansprüche**

1. Farbphotographisches Aufzeichnungsmaterial, das in mindestens einer lichtempfindlichen Silberhalogenidemulsionsschicht, einer Zwischenschicht und/oder einer Schutzschicht mindestens eine Polyalkylpiperidinverbindung als Lichtschutzmittel enthält, dadurch gekennzeichnet, dass die Polyalkylpiperidinverbindung der Formel I

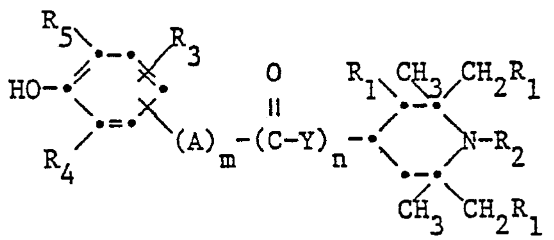

(I)

entspricht, worin

$R_1$ und $R_3$ unabhängig voneinander Wasserstoff oder Methyl sind,

$R_2$ Hydroxy, $C_1$-$C_{12}$ Alkyl, $C_3$-$C_{12}$ Alkenyl, $C_3$-$C_4$ Alkinyl, 2-Hydroxyäthyl, $C_2$-$C_{11}$ Alkoxyalkyl, $C_7$-$C_{14}$ Aralkyl oder

eine Gruppe der Formel $-(CH_2)_p-CH(R_6)-X_1$ oder $-CH(R_6)-X_2$ ist, worin p die Zahlen 1, 2 oder 3 bedeutet, $X_1$ Halogen, Cyano, $-OR_7$, $-OC(O)R_7$, $-OC(O)N(R_7)(R_8)$, $-C(O)OR_7$, $-C(O)N(R_7)(R_8)$ ist, $X_2$ Halogen, Cyano, 1,2-Epoxyäthyl, $-C(O)OR_7$, $-C(O)N(R_7)(R_8)$ und $R_6$ Wasserstoff, Methyl oder Phenyl sind, wobei $R_7$ Wasserstoff, $C_1$-$C_{12}$ Alkyl, $C_2$-$C_{12}$ Alkenyl, $C_3$-$C_{12}$ Cycloalkyl, Phenyl, $C_7$-$C_{14}$ Alkaryl, $C_7$-$C_{14}$ Aralkyl und $R_8$ Wasserstoff oder $C_1$-$C_4$ Alkyl bedeuten oder $R_7$ und $R_8$ zusammen mit dem Stickstoffatom an dem sie gebunden sind einen 5- oder 6-gliedrigen Heterocyclus bilden, oder $R_2$ eine Gruppe der Formel (II)

$$L-CO- \qquad (II)$$

bedeutet, worin L Wasserstoff, $C_1$-$C_{12}$ Alkyl, $C_2$-$C_{12}$ Alkenyl, $C_3$-$C_4$ Alkinyl, $C_3$-$C_{12}$ Cycloalkyl, $C_7$-$C_{14}$ Alkaryl, $C_7$-$C_{14}$ Aralkyl, Chlormethyl, unsubstituiertes oder durch zwei $C_1$-$C_4$ Alkyl und ein Hydroxy substituiertes Phenyl oder eine Gruppe $-OR_9$ ist, worin $R_9$ $C_1$-$C_{12}$ Alkyl, Cyclohexyl, $C_2$-$C_{12}$ Alkenyl, Benzyl oder Phenyl bedeutet, oder L eine Gruppe der Formel III

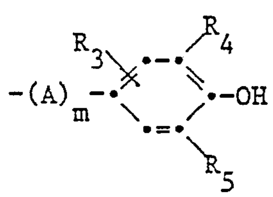

(III)

ist oder $R_2$ eine Gruppe der Formel IV

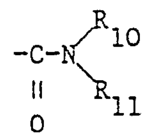

(IV)

bedeutet, worin $R_{10}$ und $R_{11}$ unabhängig voneinander $C_1$-$C_{12}$ Alkyl, $C_3$-$C_{12}$ Alkenyl, $C_3$-$C_{12}$ Cycloalkyl, Phenyl, $C_7$-$C_{14}$ Alkaryl, $C_7$-$C_{14}$ Aralkyl bedeuten und $R_{11}$ zusätzlich auch Wasserstoff sein kann, oder $R_2$ eine Gruppe der Formel V

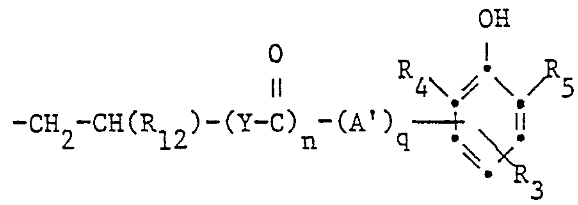

(V)

ist, worin A' Methylen oder eine Gruppe $—CH_2—CH(R_{13})—$ bedeutet und q Null oder 1 ist, und $R_{12}$ und $R_{13}$ unabhängig voneinander Wasserstoff, Methyl, Aethyl, Phenoxymethyl oder Phenyl sind, oder $R_2$ eine Gruppe der Formel VI

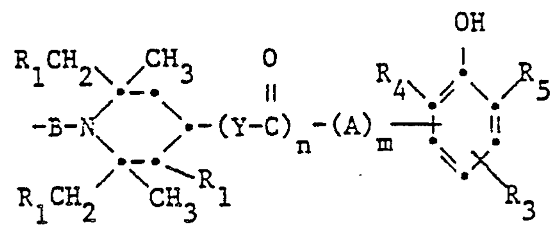

(VI)

ist, worin B eine Gruppe $C_rH_{2r}$ ist, in welcher r eine Zahl 2 bis 12 bedeutet, oder $C_4$-$C_8$ Alkenylen, $C_4$-$C_8$ Alkinylen, Phenylen, Xylylen, Bitolylen, $C_5$-$C_{12}$ Cycloalkylen oder eine Gruppe $—CONH—B_1—NHCO—$, worin $B_1$ eine Gruppe $C_rH_{2r}$ Phenylen, Naphthylen, Tolylen oder eine Gruppe der Formeln

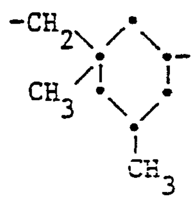

,

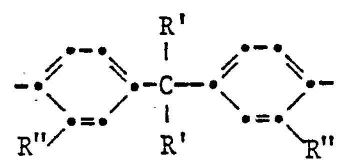

oder

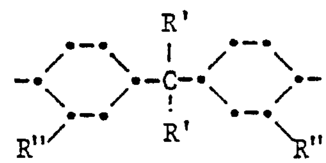

bedeutet, worin

R' Wasserstoff, Methyl oder Ethyl und

R'' Wasserstoff oder Methyl bedeuten und

r die obenangegebene Bedeutung hat,

oder $R_2$ eine der Gruppen $—S(O)_zR_{14}$ oder $—OR_{15}$ bedeutet, worin z die Zahl 1 oder 2 ist,

$R^{14}$ $C_1$-$C_{12}$ Alkyl, $C_7$-$C_{14}$ Alkaryl oder Phenyl ist und $R_{15}$ $C_1$-$C_{12}$ Alkyl, $C_3$-$C_{12}$ Alkenyl, $C_7$-$C_{14}$ Aralkyl oder eine Gruppe der Formel L'—CO— bedeutet, worin L' die oben für L angegebene Bedeutung hat,

$R_4$ und $R_5$ unabhängig voneinander $C_1$-$C_{12}$ Alkyl, $C_3$-$C_{12}$ Cycloalkyl, $C_7$-$C_{14}$ Aralkyl, $C_7$-$C_{14}$ Alkaryl oder Phenyl bedeuten und $R_4$ zusätzlich Wasserstoff oder eine Gruppe der Formel Ia

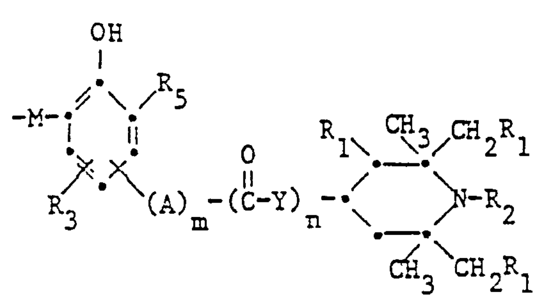

(Ia)

bedeutet, worin $R_1$, $R_2$, $R_3$, $R_5$, A, Y, m und n die angegebene Bedeutung haben und

M eine direkte Bindung, 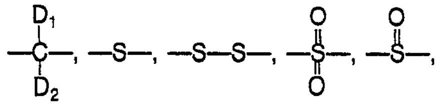, $—CH_2—S—CH_2—$, $—CH_2—O—CH_2—$, —O—, $—N(D_3)—$ ist und $D_1$ und $D_2$ unabhängig voneinander Wasserstoff, $C_1$-$C_{18}$ Alkyl, durch 1 bis 3 —S— unterbrochenes Alkyl, Phenyl bedeuten oder $D_1$ und $D_2$ zusammen mit dem sie bindenden C-Atom einen 5- oder 6-gliedrigen aliphatischen Ring bilden und $D_3$ Wasserstoff, $C_1$-$C_{18}$ Alkyl oder Phenyl ist,

Y —O— oder $—N(R_{16})—$ ist, worin $R_{16}$ Wasserstoff, $C_1$-$C_{18}$ Alkyl, $C_3$-$C_{12}$ Alkenyl, $C_3$-$C_{12}$ Cycloalkyl, Phenyl, $C_7$-$C_{14}$ Alkaryl, $C_7$-$C_{14}$ Aralkyl, $C_3$-$C_{11}$ Alkoxyalkyl,
eine Gruppe der Formel VII

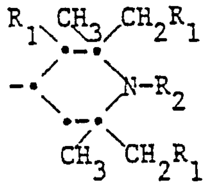

(VII)

oder eine Gruppe —Z—D ist, worin Z eine unsubstituierte oder durch eine Methylgruppe substituierte $—(CH_2)_s$-Gruppe ist, wobei s die Zahlen 2 bis 4 bedeutet und D Hydroxy, $—OR_{17}$ oder $—N(R_{18})(R_{19})$ ist, worin $R_{17}$ und $R_{18}$ Wasserstoff, $C_1$-$C_{12}$ Alkyl, $C_3$-$C_{12}$ Cycloalkyl oder eine Gruppe der Formel VIII

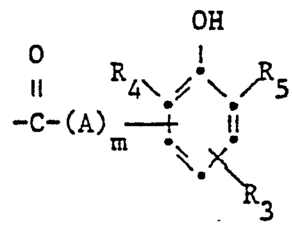

(VIII)

sind und $R_{17}$ zusätzlich auch eine Gruppe der Formel VII sein kann, $R_{19}$ Wasserstoff, $C_1$-$C_{12}$ Alkyl, $C_3$-$C_{12}$ Cycloalkyl oder eine Gruppe der Formel IX

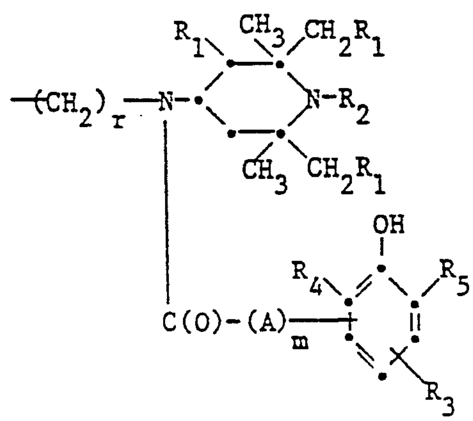

(IX)

ist oder $R_{18}$ und $R_{19}$ zusammen mit dem Stickstoffatom an dem sie gebunden sind einen 5- oder 6-gliedrigen Heterocyclus bilden, oder $R_{16}$ eine Gruppe der Formel IXa

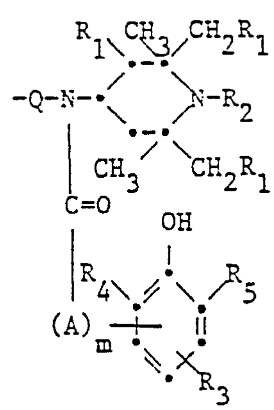

(IXa)

ist, worin Q die oben für B angegebene Bedeutung hat,

n und m unabhängig voneinander Null oder 1 bedeuten,

A Methylen oder eine Gruppe $-CH_2-CH(R_{20})-$ oder $-CH_2-C(E)(G)-$ ist, worin $R_{20}$ Wasserstoff, Methyl, Aethyl, Phenoxymethyl oder Phenyl ist, E Cyano oder eine Gruppe $-C(O)OR_{21}$, $-C(O)R_{22}$, $-SO_2R_{22}$, $-P(O)-(OR_{23})_2$, $-C(O)NR_{24}R_{25}$, $-CHO$ ist, worin $R_{21}$ $C_1$-$C_4$ Alkyl, $R_{22}$ $C_1$-$C_{12}$ Alkyl, $C_7$-$C_{14}$ Alkaryl, Phenyl, $R_{23}$ $C_1$-$C_{18}$ Alkyl, Phenyl oder Allyl, $R_{24}$ und $R_{25}$ unabhängig voneinander Wasserstoff, $C_1$-$C_{18}$ Alkyl, Phenyl bedeuten und G Wasserstoff, $C_1$-$C_{18}$ Alkyl, $C_3$-$C_{12}$ Alkenyl, $C_3$-$C_4$ Alkinyl, $C_5$-$C_{12}$ Cycloalkyl, $C_6$-$C_{18}$ Alkylcycloalkyl, $C_6$-$C_{14}$ Cycloalkylalkyl, $C_7$-$C_{14}$ Aralkyl, $C_7$-$C_{19}$ Alkylaralkyl, Phenyl, durch Phenoxy, $C_7$-$C_{10}$ Alkylphenoxy, Benzyloxy, Cyclohexyloxy, Cyano, $-COOR_{26}$, $-OCOR_{27}$ oder $-P(O)(OR_{28})_2$ substituiertes $C_1$-$C_{18}$ Alkyl ist, wobei $R_{26}$ $C_1$-$C_{18}$ Alkyl, $C_3$-$C_{12}$ Cycloalkyl oder eine Gruppe der Formel VII, $R_{27}$ $C_1$-$C_{18}$ Alkyl, $C_3$-$C_{12}$ Cycloalkyl, Phenyl, $C_7$-$C_{14}$ Aralkyl oder eine Gruppe der Formel III, $R_{28}$ $C_1$-$C_{18}$ Alkyl, Allyl oder Phenyl bedeuten, oder G durch $-O-$, $-S-$, $-SO-$, $-SO_2-$ unterbrochenes $C_2$-$C_{18}$ Alkyl,

eine Gruppe der Formel VII oder eine Gruppe der Formel

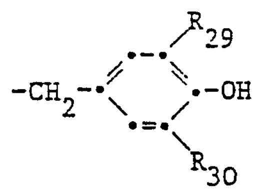

ist, worin $R_{29}$ und $R_{30}$ unabhängig voneinander $C_1$-$C_4$ Alkyl bedeuten und $R_{30}$ zusätzlich auch Wasserstoff sein kann, oder, wenn E eine Gruppe $-C(O)R_{22}$ ist, G und $R_{22}$ zusammen Trimethylen oder Tetramethylen bedeuten.

2. Farbphotographisches Aufzeichnungsmaterial gemäss Anspruch 1, dadurch gekennzeichnet, dass es als Lichtschutzmittel mindestens eine Polyalkylpiperidinverbindung der Formel X

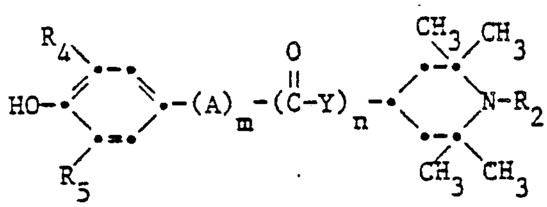

(X)

enthält, worin $R_4$ und $R_5$ unabhängig voneinander $C_1$-$C_4$ Alkyl sind und $R_4$ zusätzlich Wasserstoff oder eine Gruppe der Formel Xa

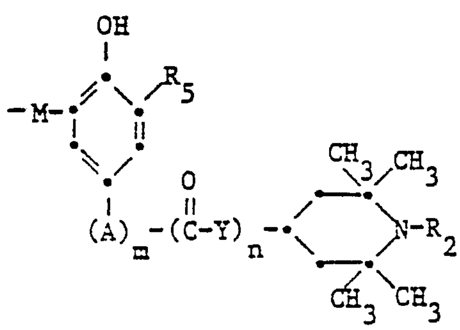

(Xa)

bedeutet, worin M eine direkte Bindung, $-CH(D_2)-$ oder $-S-$ und $D_2$ Wasserstoff oder $C_1$-$C_8$ Alkyl bedeuten,

A Methylen, Aethylen oder eine Gruppe $-CH_2-C(E)(G)-$ ist, worin E Cyano, $-COCH_3$ und $-COOCH_3$ und G Wasserstoff, $C_1$-$C_{18}$ Alkyl, Allyl, Cyclohexyl, Benzyl oder eine der beiden Gruppen der Formeln

(Siehe Formeln Seite 27 f.)

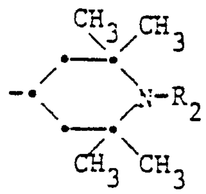 und 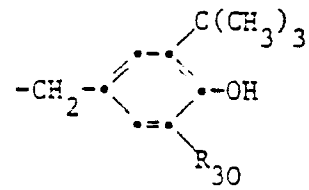

bedeuten, worin $R_{30}$ Methyl oder tert.-Butyl ist, oder E und G zusammen eine Gruppe $—CO—(CH_2)_4—$ bilden,

n und m die in Anspruch 1 angegebene Bedeutung haben,

$R_2$ Hydroxy, $C_1$-$C_4$ Alkyl, Allyl, Methallyl, Propargyl, Benzyl, 2-Hydroxyäthyl, $C_2$-$C_7$ Alkoxyalkyl, eine Gruppe der Formel $—(CH_2)_p—CH(R_6)—X_1$ oder $—CH(R_6)—X_2$ ist, worin p die Zahlen 1, 2 oder 3 bedeutet, $X_1$ Halogen, Cyano, $—OR_7$, $—OC(O)R_7$, $—OC(O)N(R_7)(R_8)$, $—C(O)R_7$, $—C(O)N(R_7)(R_8)$, $X_2$ Halogen, Cyano, 1,2-Epoxyäthyl, $—C(O)OR_7$, $—C(O)N(R_7)(R_8)$ sind und $R_6$ Wasserstoff, Methyl oder Phenyl ist, wobei $R_7$ Wasserstoff, $C_1$-$C_8$ Alkyl, Vinyl, Allyl, Methallyl, $C_5$-$C_8$ Cycloalkyl, Phenyl, $C_7$-$C_{10}$ Aralkyl und $R_8$ Wasserstoff und Methyl bedeuten, oder $R_2$ eine Gruppe der Formel II bedeutet, worin L Wasserstoff, $C_1$-$C_4$ Alkyl, Vinyl, Allyl, Cyclohexyl, Phenyl, Benzyl, Chlormethyl oder eine Gruppe $—OR_9$ ist, worin $R_9$ $C_1$-$C_8$ Alkyl, Cyclohexyl, Vinyl, Allyl oder Methallyl bedeutet oder L eine Gruppe der Formel III ist, worin m, A, $R_4$ und $R_5$ die in diesem Anspruch angegebene Bedeutung haben und $R_3$ Wasserstoff ist, oder $R_2$ eine Gruppe der Formel IV ist, worin $R_{10}$ und $R_{11}$ unabhängig voneinander $C_1$-$C_{12}$ Alkyl, Cyclohexyl und Phenyl bedeuten und $R_{11}$ zusätzlich auch Wasserstoff sein kann, oder $R_2$ eine Gruppe der Formel XI

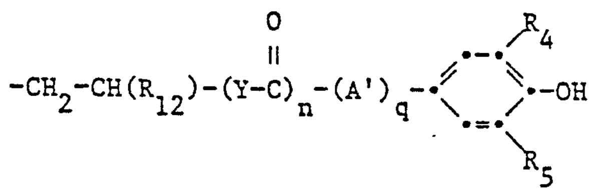 (XI)

ist, worin n, $R_4$, $R_5$ und Y die in diesem Anspruch angegebene Bedeutung haben,

A' Methylen oder eine Gruppe $—CH_2—CH(R_{13})—$ ist, q Null oder 1 bedeutet und $R_{12}$ und $R_{13}$ unabhängig voneinander Wasserstoff oder Methyl sind, oder $R_2$ eine Gruppe der Formel XII

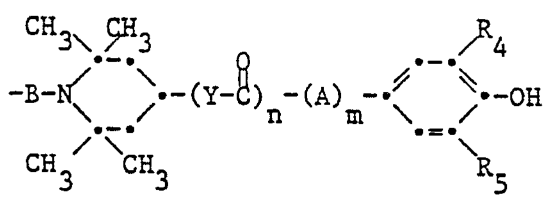 (XII)

ist, worin A, $R_4$, $R_5$, Y, n und m die in diesem Anspruch angegebene Bedeutung haben und B eine Gruppe $—(—CH_2—)_r—$ oder $—CONH—(—CH_2—)_r—NHCO—$, worin r die Zahlen 2 bis 8 bedeutet, oder $C_4$-$C_8$ Alkenylen, Xylylen oder Bitolylen ist, oder $R_2$ eine der Gruppen $—SO_2R_{14}$ oder $—OR_{15}$ bedeutet, worin $R_{14}$ $C_1$-$C_4$ Alkyl, p-Tolyl oder Phenyl ist und $R_{15}$ $C_1$-$C_4$ Alkyl, Benzyl oder eine Gruppe der Formel L'—CO— bedeutet, worin L' die in diesem Anspruch bereits für L angegebene Bedeutung hat,

Y —O— oder $—N(R_{16})—$ ist, worin $R_{16}$ Wasserstoff, $C_1$-$C_{12}$ Alkyl, Cyclohexyl, $C_3$-$C_7$ Alkoxyalkyl oder eine Gruppe der Formel XIII

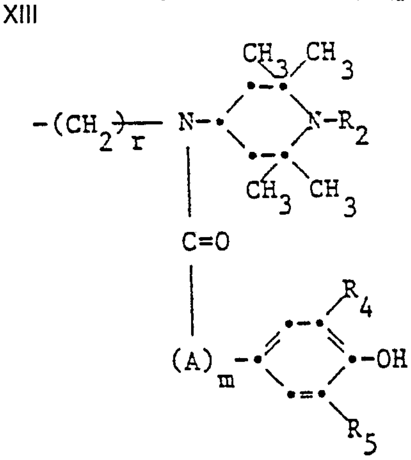 (XIII)

ist, worin r, m, A, $R_2$, $R_4$ und $R_5$ die in diesem Anspruch bereits angegebene Bedeutung haben.

3. Farbphotographisches Aufzeichnungsmaterial gemäss Anspruch 1, dadurch gekennzeichnet, dass es als Lichtschutzmittel mindestens eine Polyalkylpiperidinverbindung der Formel XIV

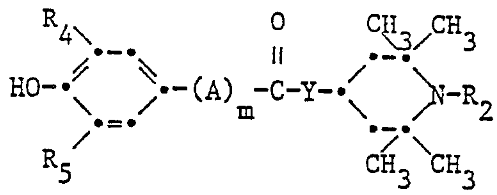

(XIV)

enthält, worin m Null oder 1 ist

A Methylen oder Aethylen bedeutet

$R_4$ und $R_5$ unabhängig voneinander Methyl oder tert.-Butyl sind

Y —O— oder —N($R_{16}$)—, worin $R_{16}$ Wasserstoff oder $C_1$-$C_8$ Alkyl ist, und

$R_2$ Hydroxy, Methyl, Allyl, Benzyl, 2-Hydroxyäthyl, Acetyl, Acryloyl, Methoxy, Acetoxy oder eine Gruppe der Formel XV

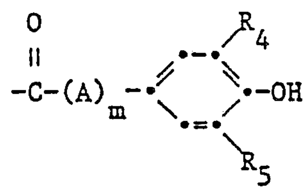

(XV)

worin m, A, $R_4$ und $R_5$ die in diesem Anspruch angegebene Bedeutung haben, oder eine Gruppe der Formel IV, worin $R_{10}$ $C_1$-$C_8$ Alkyl, Cyclohexyl oder Phenyl und $R_{11}$ Wasserstoff, $C_1$-$C_8$ Alkyl oder Cyclohexyl sind.

4. Farbphotographisches Aufzeichnungsmaterial gemäss Anspruch 1, dadurch gekennzeichnet, dass es als Lichtschutzmittel mindestens eine Polyalkylpiperidinverbindung der Formel XIV

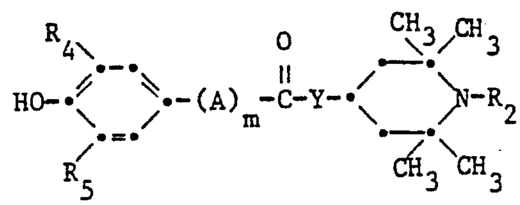

(XIV)

enthält, worin $R_2$ eine Gruppe der Formel

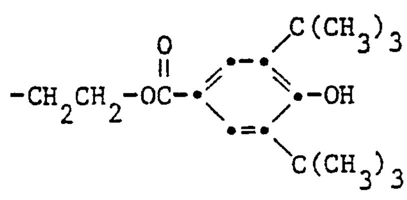

bedeutet und m, A, $R_4$, $R_5$ und Y die in Anspruch 3 angegebene Bedeutung haben.

5. Farbphotographisches Aufzeichnungsmaterial gemäss Anspruch 1, dadurch gekennzeichnet, dass es als Lichtschutzmittel mindestens eine Polyalkylpiperidinverbindung der Formel XIV

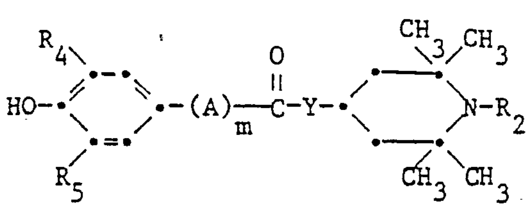

(XIV)

enthält, worin A eine Gruppe $—CH_2—C(E)(G)—$ ist, in welcher E Cyano, $—COCH_3$ und $—COOCH_3$ und G Wasserstoff oder eine der beiden Gruppen der Formeln

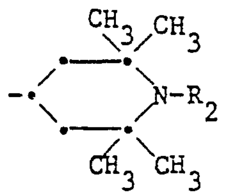

und

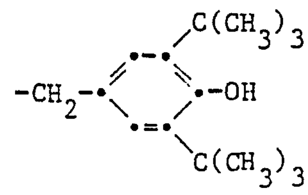

bedeuten, oder E und G zusammen eine Gruppe $—CO—(—CH_2)_4—$ bilden, $R_2$ —CHO, Acetyl oder Acryloyl bedeutet und m, $R_4$, $R_5$ und Y die in Anspruch 3 angegebene Bedeutung haben.

6. Farbphotographisches Aufzeichnungsmaterial gemäss Anspruch 1, dadurch gekennzeichnet, dass es die Lichtschutzmittel in Kombination mit Blaugrün-, Purpur- und Gelbkupplern enthält.

7. Farbphotographisches Aufzeichnungsmaterial gemäss Anspruch 1, dadurch gekennzeichnet, dass es die Lichtschutzmittel in Kombination mit Ultraviolettabsorbern enthält.

8. Farbphotographisches Aufzeichnungsmaterial gemäss Anspruch 7, dadurch gekennzeichnet, dass die Ultraviolettabsorber Verbindungen von Benzophenon-, Acrylnitril-, Thiazolidon-, Benztriazol-, Oxazol-, Thiazol- oder Imidazoltyp sind.

9. Farbphotographisches Aufzeichnungsmaterial gemäss Anspruch 1, dadurch gekennzeichnet, dass es die Lichtschutzmittel in Kombination mit Blaugrün-, Purpur- und Gelbkupplern und mit UV-Absorbern in der gleichen Schicht enthält.

10. Farbphotographisches Aufzeichnungsmaterial gemäss Anspruch 1, dadurch gekennzeichnet, dass es 1 bis 2 000 mg des Lichtschutzmittels pro $m^2$ der Schicht enthält, in die es eingearbeitet ist.

11. Verfahren zur Herstellung von photographischen Farbbildern durch bildmässige Belichtung und Farbentwicklung eines farbphotographischen Aufzeichnungsmaterials gemäss Anspruch 1.

12. Die gemäss dem Verfahren nach Anspruch 11 erhaltenen photographischen Farbbilder.

13. Verbindungen der Formel I, worin Y $—N(R_{16})—$ und n die Zahl 1 bedeuten und $R_1$, $R_2$, $R_3$, $R_4$, $R_5$, $R_{16}$, A und m die in Anspruch 1 angegebene Bedeutung haben.

14. Verbindungen gemäss Anspruch 13 der Formel X, dadurch gekennzeichnet, dass Y $—N(R_{16})—$ und n die Zahl 1 bedeuten und $R_2$, $R_4$, $R_5$, $R_{16}$, A und m die in Anspruch 2 angegebene Bedeutung haben.

15. Verbindungen gemäss Anspruch 13 der Formel XIV, dadurch gekennzeichnet, dass Y $—N(R_{16})—$ bedeutet und $R_2$, $R_4$, $R_5$, $R_{16}$, A und m die in Anspruch 3 angegebene Bedeutung haben.

## Claims

1. A colour-photographic recording material which, in at least one light-sensitive silver halide emulsion layer, an interlayer and/or a protective layer, contains at least one polyalkylpiperidine compound as a light stabiliser, wherein the polyalkylpiperidine compound is of the formula I

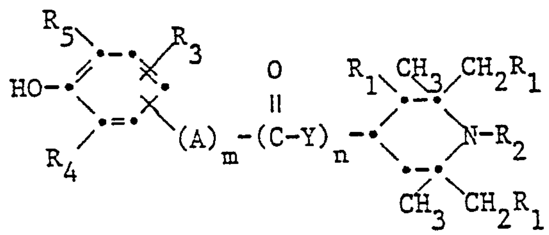

(I)

in which

each of $R_1$ and $R_3$ independently of the other is hydrogen or methyl,

$R_2$ is hydroxyl, $C_1$-$C_{12}$-alkyl, $C_3$-$C_{12}$-alkenyl, $C_3$-$C_4$-alkynyl, 2-hydroxyethyl, $C_2$-$C_{11}$-alkoxyalkyl, $C_7$-$C_{14}$-aralkyl or

a group of the formula $—(CH_2)_p—CH(R_6)—X_1$ or $—CH(R_6)—X_2$, in which p is one of the numbers 1, 2 or 3, $X_1$ is halogen, cyano, $—OR_7$, $—OC(O)R_7$, $—CC(O)N(R_7)(R_8)$, $—C(O)OR_7$ or $—C(O)N(R_7)(R_8)$, $X_2$ is halogen, cyano, 1,2-epoxyethyl, $—C(O)OR_7$ or $—C(O)N(R_7)(R_8)$ and $R_6$ is hydrogen, methyl or phenyl, $R_7$ being hydrogen, $C_1$-$C_{12}$-alkyl, $C_2$-$C_{12}$-alkenyl, $C_3$-$C_{12}$-cycloalkyl, phenyl, $C_7$-$C_{14}$-alkaryl or $C_7$-$C_{14}$-aralkyl and $R_8$ being hydrogen or $C_1$-$C_4$-alkyl, or $R_7$ and $R_8$, together with the nitrogen atom to which they are bonded, forming a 5-membered or 6-membered heterocyclic ring, or $R_2$ is a group of the formula II

L—CO— (II)

in which L is hydrogen, $C_1$-$C_{12}$-alkyl, $C_2$-$C_{12}$-alkenyl, $C_3$-$C_4$-alkynyl, $C_3$-$C_{12}$-cycloalkyl, $C_7$-$C_{14}$-alkaryl, $C_7$-$C_{14}$-aralkyl, chloromethyl, unsubstituted phenyl, phenyl which is substituted by two $C_1$-$C_4$-alkyls and one hydroxyl, or a group $—OR_9$, in which $R_9$ is $C_1$-$C_{12}$-alkyl, cyclohexyl, $C_2$-$C_{12}$-alkenyl, benzyl or phenyl, or L is a group of the formula III

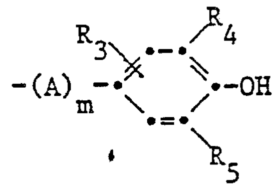 (III)

or $R_2$ is a group of the formula IV

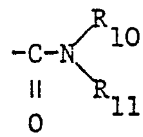 (IV)

in which each of $R_{10}$ and $R_{11}$ independently of the other is $C_1$-$C_{12}$-alkyl, $C_3$-$C_{12}$-alkenyl, $C_3$-$C_{12}$-cycloalkyl, phenyl, $C_7$-$C_{14}$-alkaryl or $C_7$-$C_{14}$-aralkyl and $R_{11}$ additionally can also be hydrogen, or $R_2$ is a group of the formula V

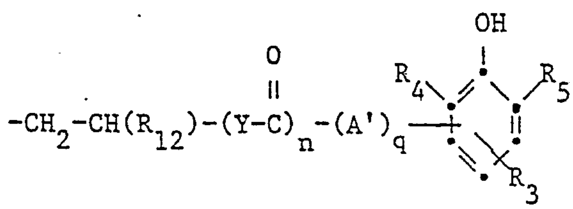 (V)

in which A' is methylene or a group $—CH_2—CH(R_{13})—$ and q is zero or 1, and each of $R_{12}$ and $R_{13}$ independently of the other is hydrogen, methyl, ethyl, phenoxymethyl or phenyl, or $R_2$ is a group of the formula VI

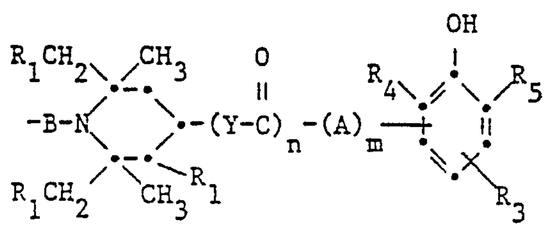 (VI)

in which B is a group $C_rH_{2r}$, in which r is a number from 2 to 12, or is $C_4$-$C_8$-alkenylene, $C_4$-$C_8$-alkynylene, phenylene, xylylene, bitolylene, $C_5$-$C_{12}$-cycloalkylene or a group $—CONH—B_1—NHCO—$, in which $B_1$ is a group $C_rH_{2r}$, phenylene, naphthylene, tolylene or a group of the formulae

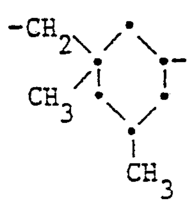 , 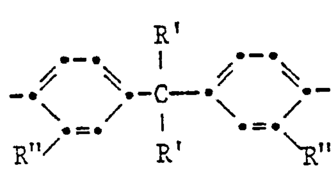 or 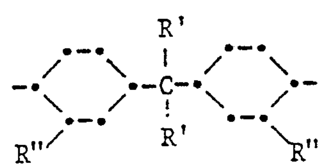

in which

R' is hydrogen, methyl or ethyl,
R'' is hydrogen or methyl and
r is as defined above,

or $R_2$ is one of the groups $—S(O)_zR_{14}$ or $—OR_{15}$, in which z is the number 1 or 2, $R_{14}$ is $C_1$-$C_{12}$-alkyl, $C_7$-$C_{14}$-alkaryl or phenyl and $R_{15}$ is $C_1$-$C_{12}$-alkyl, $C_3$-$C_{12}$-alkenyl, $C_7$-$C_{14}$-aralkyl or a group of the formula L'—CO—, in which L' is as defined above for L,

each of $R_4$ and $R_5$ independently of the other is $C_1$-$C_{12}$-alkyl, $C_3$-$C_{12}$-cycloalkyl, $C_7$-$C_{14}$-aralkyl, $C_7$-$C_{14}$ alkaryl or phenyl and $R_4$ can additionally also be hydrogen or a group of the formula Ia

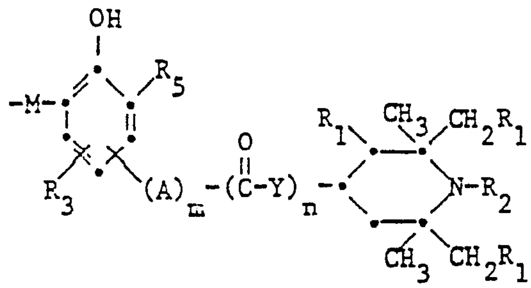

(Ia)

in which $R_1$, $R_2$, $R_3$, $R_5$, A, Y, m and n are as defined and

M is a direct bond, 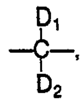, —S—, —S—S—, 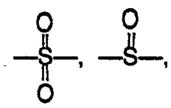, $—CH_2—S—CH_2—$, $—CH_2—O—CH_2—$, —O— and $—N(D_3)—$, and each of $D_1$ and $D_2$ independently of the other is hydrogen, $C_1$-$C_{18}$-alkyl, alkyl interrupted by 1 to 3 —S—, or phenyl, or $D_1$ and $D_2$, together with the C atom linking them, form a 5-membered or 6-membered aliphatic ring and $D_3$ is hydrogen, $C_1$-$C_{18}$-alkyl or phenyl,

Y is —O— or $—N(R_{16})—$, in which $R_{16}$ is hydrogen, $C_1$-$C_{18}$-alkyl, $C_3$-$C_{12}$-alkenyl, $C_3$-$C_{12}$-cycloalkyl, phenyl, $C_7$-$C_{14}$-alkaryl, $C_7$-$C_{14}$-aralkyl, $C_3$-$C_{11}$-alkoxyalkyl, a group of the formula VII

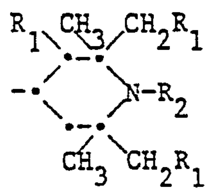

(VII)

or a group —Z—D, in which Z is a $—(CH_2)_s$ group unsubstituted or substituted by a methyl group, s being one of the numbers 2 to 4 and D being hydroxyl, $—OR_{17}$ or $—N(R_{18})(R_{19})$, in which $R_{17}$ and $R_{18}$ are hydrogen, $C_1$-$C_{12}$-alkyl, $C_3$-$C_{12}$-cycloalkyl or a group of the formula VIII

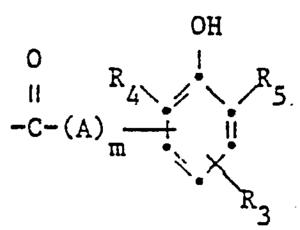

(VIII)

and $R_{17}$ additionally can also be a group of the formula VII, $R_{19}$ is hydrogen, $C_1$-$C_{12}$-alkyl, $C_3$-$C_{12}$-cycloalkyl or a group of the formula IX

(See formula IX page 32)

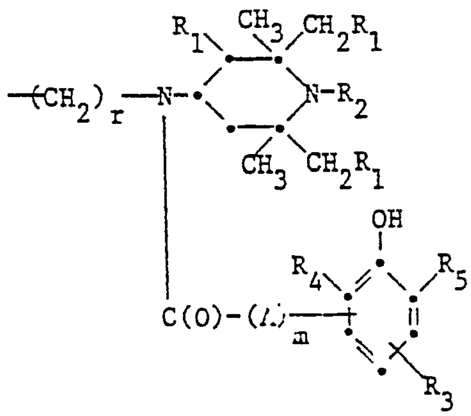

(IX)

or $R_{18}$ and $R_{19}$, together with the nitrogen atom to which they are bonded, form a 5-membered or 6-membered heterocyclic ring, or $R_{16}$ is a group of the formula IXa

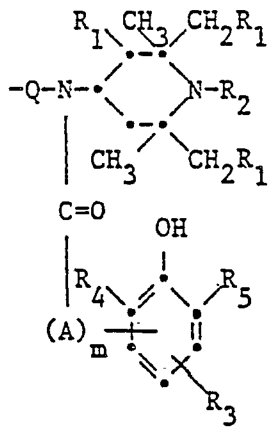

(IXa)

in which Q is as defined above for B,

each of n and m independently of the other is zero or 1, and

A is methylene or a group $-CH_2-CH(R_{20})-$ or $-CH_2-C(E)(G)-$, in which $R_{20}$ is hydrogen, methyl, ethyl, phenoxymethyl or phenyl, E is cyano or a group $-C(O)OR_{21}$, $-C(O)R_{22}$, $-SO_2R_{22}$, $-P(O)-(OR_{23})_2$, $-C(O)NR_{24}R_{25}$ or $-CHO$, in which $R_{21}$ is $C_1$-$C_4$-alkyl, $R_{22}$ is $C_1$-$C_{12}$-alkyl, $C_7$-$C_{14}$-alkaryl or phenyl, $R_{23}$ is $C_1$-$C_{18}$-alkyl, phenyl or allyl, and each of $R_{24}$ and $R_{25}$ independently of the other is hydrogen, $C_1$-$C_{18}$-alkyl or phenyl, and G is hydrogen, $C_1$-$C_{18}$-alkyl, $C_3$-$C_{12}$-alkenyl, $C_3$-$C_4$-alkynyl, $C_5$-$C_{12}$-cycloalkyl, $C_6$-$C_{18}$-alkylcycloalkyl, $C_6$-$C_{14}$-cycloalkylalkyl, $C_7$-$C_{14}$-aralkyl, $C_7$-$C_{19}$-alkylaralkyl, phenyl, or $C_1$-$C_{18}$-alkyl which is substituted by phenoxy, $C_7$-$C_{10}$-alkylphenoxy, benzyloxy, cyclohexyloxy, cyano, $-COOR_{26}$, $-OCOR_{27}$ or $-P(O)(OR_{28})_2$, $R_{26}$ being $C_1$-$C_{18}$-alkyl, $C_3$-$C_{12}$-cycloalkyl or a group of the formula VII, $R_{27}$ being $C_1$-$C_{18}$-alkyl, $C_3$-$C_{12}$-cycloalkyl, phenyl, $C_7$-$C_{14}$-aralkyl or a group of the formula III and $R_{28}$ being $C_1$-$C_{18}$-alkyl, allyl or phenyl, or G is $C_2$-$C_{18}$-alkyl interrupted by $-O-$, $-S-$, $-SO-$, or $-SO_2-$,
a group of the formula VII or a group of the formula

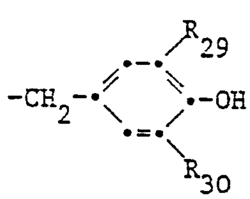

in which each of $R_{29}$ and $R_{30}$ independently of the other is $C_1$-$C_4$-alkyl and $R_{30}$ additionally can also be hydrogen, or, if E is a group $-C(O)R_{22}$, G and $R_{22}$ together are trimethylene or tetramethylene.

2. A colour-photographic recording material according to claim 1, which contains, as the light stabiliser, at least one polyalkylpiperidine compound of the formula X

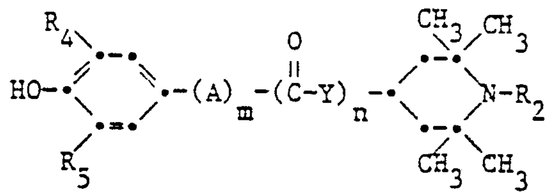

(X)

in which each of $R_4$ and $R_5$ independently of the other is $C_1$-$C_4$-alkyl and $R_4$ additionally can also be hydrogen or a group of the formula Xa

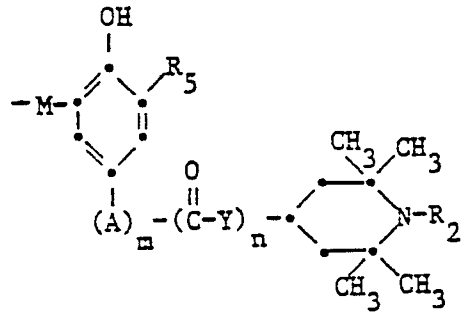

(Xa)

in which M is a direct bond, $—CH(D_2)—$ or —S— and $D_2$ is hydrogen or $C_1$-$C_8$-alkyl,

A is methylene, ethylene or a group $—CH_2—C(E)(G)—$, in which E is cyano, $—COCH_3$ or $—COOCH_3$ and G is hydrogen, $C_1$-$C_{18}$-alkyl, allyl, cyclohexyl, benzyl or one of the two groups of the formulae

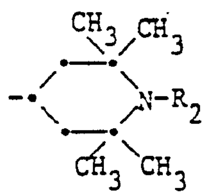

and

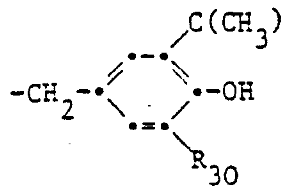

in which $R_{30}$ is methyl or tert -butyl, or E and G together form a group $—CO—(CH_2)_4—$,

n and m are as defined in claim 1,

$R_2$ is hydroxyl, $C_1$-$C_4$-alkyl, allyl, methallyl, propargyl, benzyl, 2-hydroxyethyl, $C_2$-$C_7$-alkoxyalkyl or a group of the formula $—(CH_2)_p—CH(R_6)—X_1$ or $—CH(R_6)—X_2$, in which p is one of the numbers 1, 2 or 3, $X_1$ is halogen, cyano, $—OR_7$, $—OC(O)R_7$, $—CC(O)N(R_7)(R_8)$, $—C(O)R_7$ or $—C(O)N(R_7)(R_8)$, $X_2$ is halogen, cyano, 1,2-epoxyethyl, $—C(O)OR_7$ or $—C(O)N(R_7)(R_8)$ and $R_6$ is hydrogen, methyl or phenyl, $R_7$ being hydrogen, $C_1$-$C_8$-alkyl, vinyl, allyl, methallyl, $C_5$-$C_8$-cycloalkyl, phenyl or $C_7$-$C_{10}$-aralkyl and $R_8$ being hydrogen or methyl, or $R_2$ is a group of the formula II, in which L is hydrogen, $C_1$-$C_4$-alkyl, vinyl, allyl, cyclohexyl, phenyl, benzyl, chloromethyl or a group $—OR_9$, in which $R_9$ is $C_1$-$C_8$-alkyl, cyclohexyl, vinyl, allyl or methallyl, or L is a group of the formula III, in which m, A, $R_4$ and $R_5$ are as defined in this claim, and $R_3$ is hydrogen, or $R_2$ is a group of the formula IV, in which each of $R_{10}$ and $R_{11}$ independently of the other is $C_1$-$C_{12}$-alkyl, cyclohexyl or phenyl and $R_{11}$ additionally can also be hydrogen, or $R_2$ is a group of the formula XI

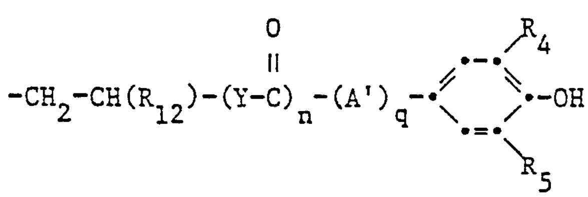

(XI)

in which n, $R_4$, $R_5$ and Y are as defined in this claim,

A' is methylene or a group $—CH_2—CH(R_{13})—$, q is zero or 1, and each of $R_{12}$ and $R_{13}$ independently of the other is hydrogen or methyl, or $R_2$ is a group of the formula XII

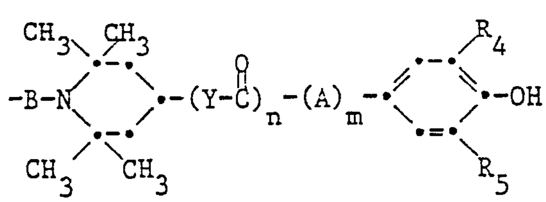

(XII)

in which A, $R_4$, $R_5$, Y, n and m are as defined in this claim and B is a group $-(-CH_2-)_r-$ or $-CONH-(-CH_2-)_r-NHCO-$, in which r is one of the numbers 2 to 8, or $C_4$-$C_8$-alkenylene, xylylene or bitolylene, or $R_2$ is one of the groups $-SO_2R_{14}$ or $-OR_{15}$, in which $R_{14}$ is $C_1$-$C_4$-alkyl, p-tolyl or phenyl and $R_{15}$ is $C_1$-$C_4$-alkyl, benzyl or a group of the formula L'—CO—, in which L' is as already defined for L in this claim, and

Y is —O— or $-N(R_{16})-$, in which $R_{16}$ is hydrogen, $C_1$-$C_{12}$-alkyl, cyclohexyl, $C_3$-$C_7$-alkoxyalkyl or a group of the formula XIII

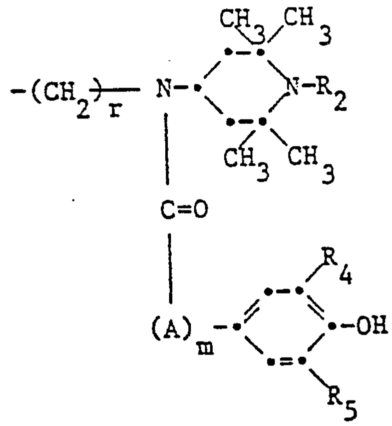

(XIII)

in which r, m, A, $R_2$, $R_4$ and $R_5$ are as already defined in this claim.

3. A colour-photographic recording material according to claim 1, which contains, as the light stabiliser, at least one polyalkylpiperidine compound of the formula XIV

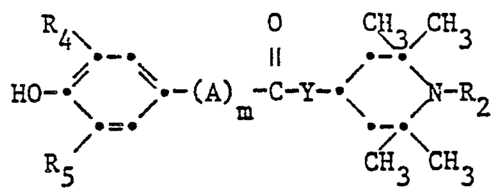

(XIV)

in which m is zero or 1,

A is methylene or ethylene, each of

$R_4$ and $R_5$ independently of the other is methyl or tert-butyl,

Y is —O— or $-N(R_{16})-$, in which $R_{16}$ is hydrogen or $C_1$-$C_8$-alkyl, and

$R_2$ is hydroxyl, methyl, allyl, benzyl, 2-hydroxyethyl, acetyl, acryloyl, methoxy, acetoxy or a group of the formula XV

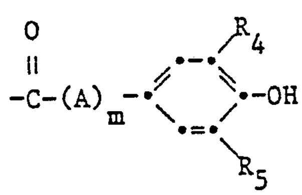

(XV)

in which m, A, $R_4$ and $R_5$ are as defined in this claim, or is a group of the formula IV, in which $R_{10}$ is $C_1$-$C_8$-alkyl, cyclohexyl or phenyl and $R_{11}$ is hydrogen, $C_1$-$C_8$-alkyl or cyclohexyl.

4. A colour-photographic recording material according to claim 1, which contains, as the light stabiliser, at least one polyalkylpiperidine compound of the formula XIV

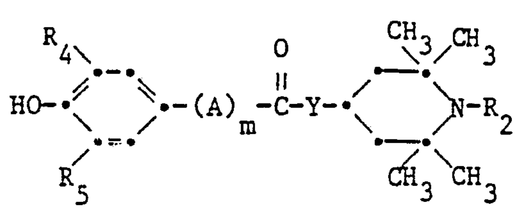

(XIV)

in which $R_2$ is a group of the formula

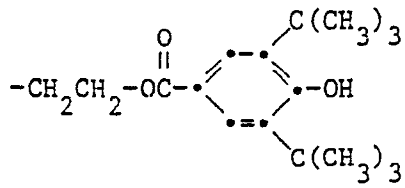

and m, A, $R_4$, $R_5$ and Y are as defined in claim 3.

5. A colour-photographic recording material according to claim 1, which contains, as the light stabiliser, at least one polyalkylpiperidine compound of the formula XIV

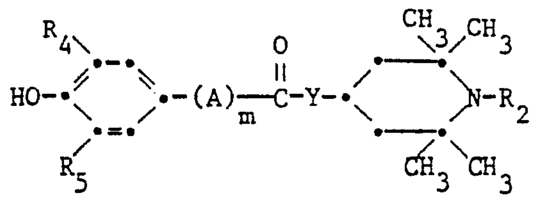

(XIV)

in which A is a group $—CH_2—C(E)(G)—$, in which E is cyano, $—COCH_3$ or $—COOCH_3$ and G is hydrogen or one of the two groups of the formulae

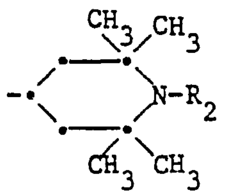

and

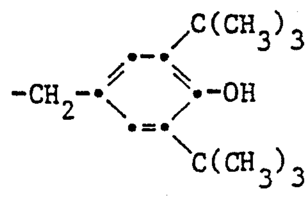

or E and G together form a group $—CO—(—CH_2)_4—$, $R_2$ is —CHO, acetyl or acryloyl and m, $R_4$, $R_5$ and Y are as defined in claim 3.

6. A colour-photographic recording material according to claim 1, which contains the light stabiliser in combination with a cyan coupler, magenta coupler and yellow coupler.

7. A colour-photographic recording material according to claim 1, which contains the light stabiliser in combination with an ultraviolet absorber.

8. A colour-photographic recording material according to claim 7, wherein the ultraviolet absorber is a compound of the benzophenone, acrylonitrile, thiazolidone, benzotriazole, oxazole, thiazole or imidazole type.

9. A colour-photographic recording material according to claim 1, which contains the light stabiliser in combination with a cyan coupler, magenta coupler and yellow coupler and with a UV absorber in the same layer.

10. A colour-photographic recording material according to claim 1, which contains 1 to 2,000 mg of the light stabiliser per $m^2$ of the layer into which the latter is incorporated.

11. A process for the production of photographic colour images by imagewise exposure and colour development of a colour-photographic recording material according to claim 1.

12. A photographic colour image obtained by the process according to claim 11.

13. A compound of the formula I, in which Y is $—N(R_{16})—$ and n is the number 1, and $R_1$, $R_2$, $R_3$, $R_4$, $R_5$, $R_{16}$, A and m are as defined in claim 1.

14. A compound according to claim 13, of the formula X, wherein Y is $—N(R_{16})—$ and n is the number 1, and $R_2$, $R_4$, $R_5$, $R_{16}$, A and m are as defined in claim 2.

15. A compound according to claim 13, of the formula XIV, wherein Y is $—N(R_{16})—$ and $R_2$, $R_4$, $R_5$, $R_{16}$, A and m are as defined in claim 3.

**Revendications**

1. Matière d'enregistrement pour photographie en couleurs qui contient, comme stabilisant à la lumière, au moins un composé polyalkyl-pipéridinique dans au moins une couche d'émulsion d'halogénure d'argent photosensible, une couche intermédiaire et/ou une couche protectrice, matière caractérisée en ce que le composé polyalkyl-pipéridinique répond à la formule I :

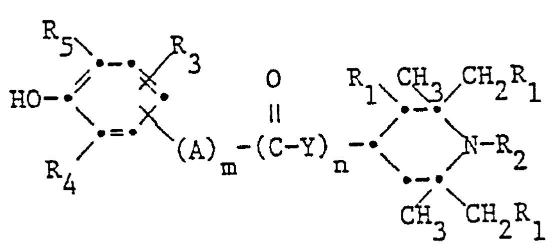

(I)

dans laquelle

$R_1$ et $R_3$ représentent chacun, indépendamment l'un de l'autre, l'hydrogène ou un méthyle,

$R_2$ représente un hydroxy, un alkyle en $C_1$ à $C_{12}$, un alcényle en $C_3$-$C_{12}$, un alcynyle en $C_3$ ou $C_4$, un hydroxy-2 éthyle, un alcoxyalkyle en $C_2$-$C_{11}$, un aralkyle en $C_7$-$C_{14}$ ou un radical répondant à l'une des formules : $-(CH_2)_p-CH(R_6)-X_1$ et $-CH(R_6)-X_2$ dans lesquelles p désigne un nombre égal à 1, à 2 ou à 3, $X_1$ représente un halogène ou un radical cyano, $-OR_7$, $-OC(O)R_7$, $-OC(O)N(R_7)(R_8)$, $-C(O)OR-$, ou $-C(O)N(R_7)(R_8)$, $X_2$ représente un halogène ou un radical cyano, époxy-1,2 éthyle, $-C(O)OR_7$, $-C(O)N(R_7)(R_8)$ et $R_6$ représente l'hydrogène ou un radical méthyle ou phényle, $R_7$ désignant l'hydrogène, un alkyle en $C_1$-$C_{12}$, un alcényle en $C_2$-$C_{12}$, un cycloalkyle en $C_3$-$C_{12}$, un phényle, un alkylaryle en $C_7$-$C_{14}$ ou un aralkyle en $C_7$-$C_{14}$ et $R_8$ l'hydrogène ou un alkyle en $C_1$-$C_4$, ou encore $R_7$ et $R_8$ formant ensemble et avec l'atome d'azote auquel ils sont liés un hétérocycle à 5 ou 6 maillons, ou $R_2$ représente un radical répondant à la formule II :

$$L-CO- \qquad (II)$$

dans laquelle L représente l'hydrogène, un alkyle en $C_1$-$C_{12}$, un alcényle en $C_2$-$C_{12}$, un alcynyle en $C_3$ ou $C_4$, un cycloalkyle en $C_3$-$C_{12}$, un alkylaryle en $C_7$-$C_{14}$, un aralkyle en $C_7$-$C_{14}$, un chlorométhyle, un phényle non substitué ou porteur de 2 alkyles en $C_1$-$C_4$ et d'un hydroxy, ou un radical $-OR_9$ dans lequel $R_9$ désigne un alkyle en $C_1$-$C_{12}$, un cyclohexyle, un alcényle en $C_2$-$C_{12}$, un benzyle ou un phényle, ou L représente un radical de formule III :

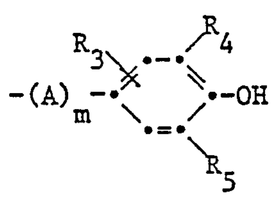

(III)

ou $R_2$ représente un radical répondant à la formule IV :

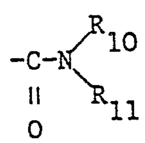

(IV)

dans laquelle $R_{10}$ et $R_{11}$ représentent chacun, indépendamment l'un de l'autre, un alkyle en $C_1$-$C_{12}$, un alcényle en $C_3$-$C_{12}$, un cycloalkyle en $C_3$-$C_{12}$, un phényle, un alkylaryle en $C_7$-$C_{14}$ ou un aralkyle en $C_7$-$C_{14}$, $R_{11}$ pouvant en outre représenter l'hydrogène, ou $R_2$ représente un radical répondant à la formule V :

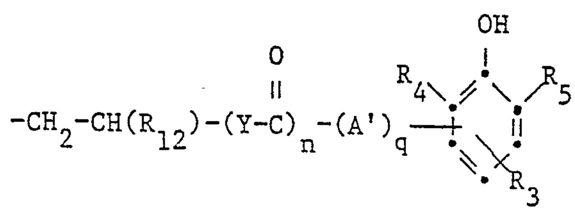

(V)

dans laquelle A' représente un méthylène ou un radical $-CH_2-CH(R_{13})-$, q est égal à 0 ou à 1, et $R_{12}$ et $R_{13}$ représentent chacun, indépendamment l'un de l'autre, l'hydrogène ou un radical méthyle, éthyle, phénoxyméthyle ou phényle, ou $R_2$ représente un radical répondant à la formule VI :

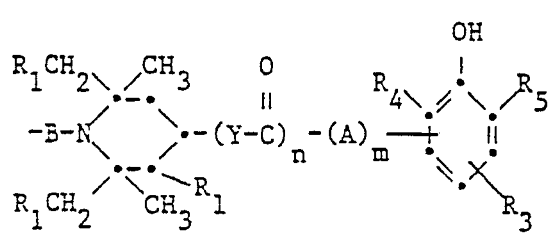

(VI)

dans laquelle B représente un radical $C_rH_{2r}$ dont l'indice r désigne un nombre de 2 à 12, ou représente un alcénylène en $C_4$-$C_8$, un alcynylène en $C_4$-$C_8$, un phénylène, un xylylène, un bitolylène, un cycloalkylène en $C_5$-$C_{12}$ ou un radical —CONH—$B_1$—NHCO— dans lequel $B_1$ représente un radical $C_rH_{2r}$, phénylène, naphtylène ou tolylène ou un radical répondant à l'une des formules :

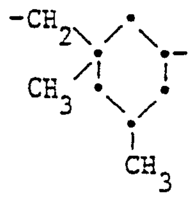 , 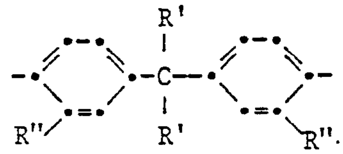 ou 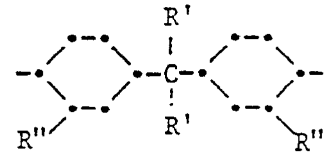

dans lesquelles

R' représente l'hydrogène, un méthyle ou un éthyle,

R" représente l'hydrogène ou un méthyle et

r a la signification précédemment donnée,

ou $R_2$ représente un radical répondant à l'une des formules : —$S(O)_zR_{14}$ et —$OR_{15}$ dans lesquelles

$R^{14}$ représente un alkyle en $C_1$-$C_{12}$, un aralkyle en $C_7$-$C_{14}$ ou un phényle et $R_{15}$ représente un alkyle en $C_1$-$C_{12}$, un alcényle en $C_3$-$C_{12}$, un aralkyle en $C_7$-$C_{14}$ ou un radical L'—CO— dans lequel L' a la signification qui a été donnée ci-dessus pour L,

$R_4$ et $R_5$ représentent chacun, indépendamment l'un de l'autre, un alkyle en $C_1$-$C_{12}$, un cycloalkyle en $C_3$-$C_{12}$, un aralkyle en $C_7$-$C_{14}$, un alkylaryle en $C_7$-$C_{14}$ ou un phényle, $R_4$ pouvant en outre représenter l'hydrogène ou un radical répondant à la formule Ia :

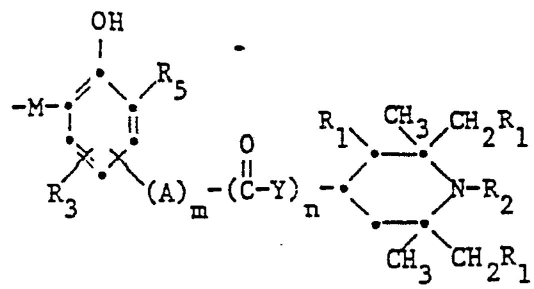 (Ia)

dans laquelle $R_1$, $R_2$, $R_3$, $R_5$, A, Y, m et n ont les significations précédemment données,

M représente une liaison directe ou un radical 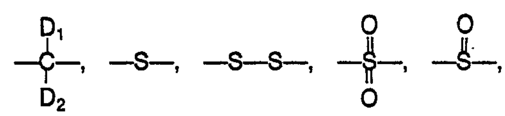

—$CH_2$—S—$CH_2$—, —$CH_2$—O—$CH_2$—, —O— ou —N($D_3$)—, les symboles $D_1$ et $D_2$ représentent chacun, indépendamment l'un de l'autre, l'hydrogène, un alkyle en $C_1$-$C_{18}$, un alkyle interrompu par 1 à 3 ponts —S— ou un phényle, ou bien les deux forment ensemble et avec l'atome de carbone auquel ils sont liés un noyau aliphatique pentagonal ou hexagonal, et $D_3$ représente l'hydrogène, un alkyle en $C_1$-$C_{18}$ ou un phényle,

Y représente —O— ou un radical —N($R_{16}$) dans lequel $R_{16}$ représente l'hydrogène, un alkyle en $C_1$-$C_{18}$, un alcényle en $C_3$-$C_{12}$, un cycloalkyle en $C_3$-$C_{12}$, un phényle, un alkylaryle en $C_7$-$C_{14}$, un aralkyle en $C_7$-$C_{14}$, un alcoxyalkyle en $C_3$-$C_{11}$,

un radical de formule VII :

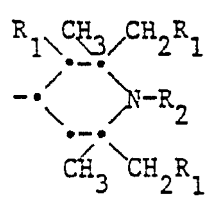 (VII)

ou un radical —Z—D dans lequel Z représente un radical —$(CH_2)_s$— non substitué ou porteur d'un méthyle, l'indice s désignant un nombre de 1 à 4, et D représente un radical hydroxy, —$OR_{17}$ ou —N($R_{18}$)($R_{19}$), où $R_{17}$ et $R_{18}$ représentent chacun l'hydrogène, un alkyle en $C_1$-$C_{12}$, un cycloalkyle en $C_3$-$C_{12}$ ou un radical de formule VIII :

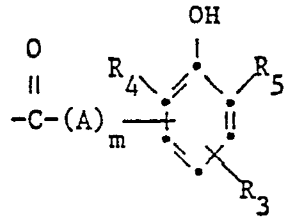

(VIII)

et $R_{17}$ peut en outre représenter aussi un radical de formule VII, $R_{19}$ représente l'hydrogène, un alkyle en $C_1$-$C_{12}$, un cycloalkyle en $C_3$-$C_{12}$ ou un radical de formule IX :

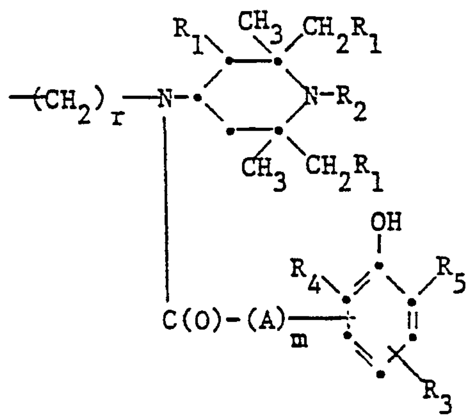

(IX)

ou $R_{18}$ et $R_{19}$ forment ensemble, et avec l'atome d'azote qui les unit, un hétérocycle à 5 ou 6 maillons, ou $R_{16}$ représente un radical répondant à la formule IXa :

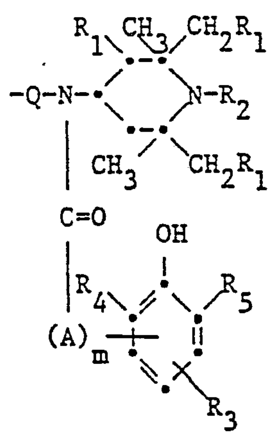

(IXa)

dans laquelle Q a la signification qui a été donnée ci-dessus pour B tandis que

n et m représentent chacun, indépendamment l'un de l'autre, le nombre 0 ou le nombre 1, et

A représente un radical méthylène ou un radical répondant à l'une des formules : $—CH_2—CH(R_{20})$ et $—CH_2—C(E)(G)—$ dans lesquelles $R_{20}$ représente l'hydrogène ou un radical méthyle, éthyle, phénoxyméthyle ou phényle, E représente un cyano ou l'un des radicaux $—C(O)OR_{21}$, $—C(O)R_{22}$, $—SO_2R_{22}$, $—P(O)—(OR_{23})_2$, $—C(O)NR_{24}R_{25}$ et —CHO, dans lesquels $R_{21}$ représente un alkyle en $C_1$-$C_4$, $R_{22}$ un alkyle en $C_1$-$C_{12}$, un alkylaryle en $C_7$-$C_{14}$ ou un phényle, $R_{23}$ un alkyle en $C_1$-$C_{18}$, un phényle ou un allyle, et $R_{24}$ et $R_{25}$ représentent chacun, indépendamment l'un de l'autre, l'hydrogène, un alkyle en $C_1$-$C_{18}$ ou un phényle, et G représente l'hydrogène, un alkyle en $C_1$-$C_{18}$, un alcényle en $C_3$-$C_{12}$, un alcynyle en $C_3$ ou $C_4$, un cycloalkyle en $C_5$-$C_{12}$, un alkylcycloalkyle en $C_6$-$C_{18}$, un cycloalkyl-alkyle en $C_6$-$C_{14}$, un aralkyle en $C_7$-$C_{14}$, un alkyl-aralkyle en $C_7$-$C_{19}$, un phényle ou un alkyle en $C_1$-$C_{18}$ porteur d'un phénoxy, d'un alkylphénoxy en $C_7$-$C_{10}$, d'un benzyloxy, d'un cyclohexyloxy, d'un cyano ou d'un radical $—COOR_{26}$, $—OCOR_{27}$ ou $—P(O)(OR_{28})_2$ où $R_{26}$ représente un alkyle en $C_1$-$C_{18}$, un cycloalkyle en $C_3$-$C_{12}$ ou un radical de formule VII, $R_{27}$ représente un alkyle en $C_1$-$C_{18}$, un cycloalkyle en $C_3$-$C_{12}$, un phényle, un aralkyle en $C_7$-$C_{14}$ ou un radical de formule III et $R_{28}$ représente un alkyle en $C_1$-$C_{18}$, un allyle ou un phényle, ou G représente un alkyle interrompu par —O—, —S—, —SO— ou $—SO_2—$, un radical de formule VII

ou un radical répondant à la formule suivante :

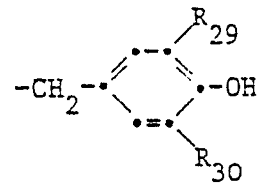

dans laquelle $R_{29}$ et $R_{30}$ représentent chacun, indépendamment l'un de l'autre, un alkyle en $C_1$-$C_4$, et $R_{30}$ peut en outre représenter l'hydrogène, ou encore, lorsque E désigne un radical —C(O)$R_{22}$, G et $R_{22}$ forment ensemble un radical triméthylène ou tétraméthylène.

2. Matière d'enregistrement pour photographie en couleurs selon la revendication 1, matière caractérisée en ce qu'elle contient, comme stabilisant à la lumière, au moins un composé polyalkylpipéridinique répondant à la formule X :

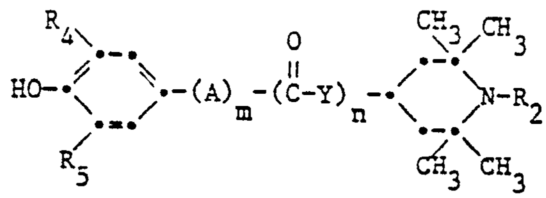

(X)

dans laquelle $R_4$ et $R_5$ représentent chacun, indépendamment l'un de l'autre, un alkyle en $C_1$-$C_4$, $R_4$ pouvant en outre représenter l'hydrogène ou un radical répondant à la formule Xa :

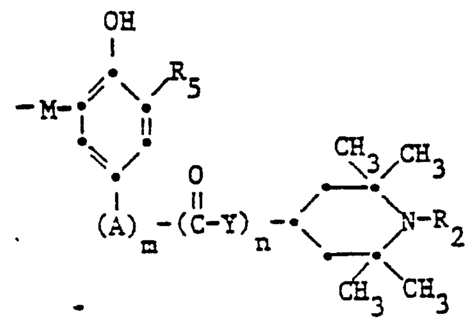

(Xa)

dans laquelle M représente une liaison directe, —CH($D_2$)— ou —S— et $D_2$ représente l'hydrogène ou un alkyle en $C_1$-$C_8$,

A représente un radical méthylène, un radical éthylène ou un radical —$CH_2$—C(E)(G)— dans lequel E représente un radical cyano, —$COCH_3$ ou —$COOCH_3$ et G l'hydrogène, un alkyle en $C_1$-$C_{18}$, un allyle, un cyclohexyle, un benzyle ou un radical répondant à l'une des deux formules suivantes :

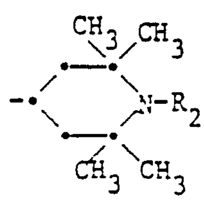

et

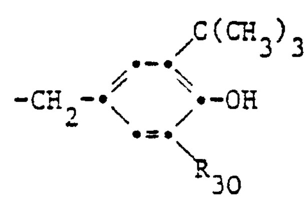

(où $R_{30}$ représente un radical méthyle ou tert-butyle), ou E et G forment ensemble un radical —CO—$(CH_4)_4$—,

n et m ont les significations données à la revendication 1,

$R_2$ représente un hydroxy, un alkyle en $C_1$-$C_4$, un allyle, un méthallyle, un propyne-2 yle, un benzyle, un hydroxy-2 éthyle, un alcoxy-alkyle en $C_2$-$C_7$,

ou un radical répondant à l'une des formules : —$(CH_2)_p$—CH($R_6$)—$X_1$ et —CH($R_6$)—$X_2$ dans lesquelles p représente un nombre égal à 1, à 2 ou à 3, $X_1$ représente un halogène ou un radical cyano, —$OR_7$, —OC(O)$R_7$, —OC(O)N($R_7$)($R_8$), —C(O)$R_7$, —C(O)N($R_7$)($R_8$), $X_2$ représente un halogène ou un radical cyano, époxy-1,2 éthyle, —C(O)O$R_7$, —C(O)N($R_7$)($R_8$) et $R_6$ représente l'hydrogène, un méthyle ou un phényle, le symbole $R_7$ désignant l'hydrogène, un alkyle en $C_1$-$C_8$, un vinyle, un allyle, un méthallyle, un cycloalkyle en $C_5$-$C_8$, un phényle ou un aralkyle en $C_7$-$C_{10}$ et $R_8$ l'hydrogène ou un méthyle, ou $R_2$ représente un radical de formule II dans lequel L représente l'hydrogène, un alkyle en $C_1$-$C_4$, un vinyle, un

allyle, un cyclohexyle, un phényle, un benzyle, un chlorométhyle ou un radical —$OR_9$ dont le symbole $R_9$ désigne un alkyle en $C_1$-$C_8$, un cyclohexyle, un vinyle, un allyle ou un méthallyle, ou L représente un radical de formule III dans lequel m, A, $R_4$ et $R_5$ ont les significations qui ont été données ci-dessus dans cette revendication et $R_3$ représente l'hydrogène, ou $R_2$ représente un radical de formule IV dans lequel $R_{10}$ et $R_{11}$ représentent chacun, indépendamment l'un de l'autre, un alkyle en $C_1$-$C_{12}$, un cyclohexyle ou un phényle, $R_{11}$ pouvant en outre représenter l'hydrogène, ou $R_2$ représente un radical répondant à la formule XI :

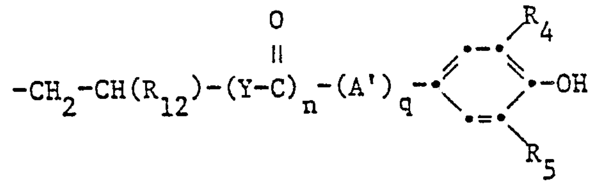

(XI)

dans laquelle n, $R_4$, $R_5$ et Y ont les significations qui ont été données ci-dessus dans cette même revendication,

A' représente un méthylène ou un radical —$CH_2$—$CH(R_{13})$—, q représente le nombre 0 ou le nombre 1 et $R_{12}$ et $R_{13}$ représentent chacun, indépendamment l'un de l'autre, l'hydrogène ou un méthyle, ou $R_2$ représente un radical répondant à la formule XII :

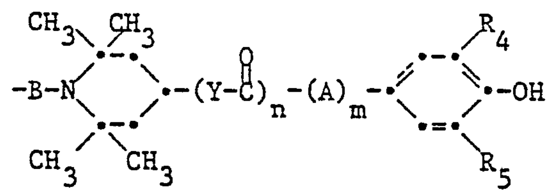

(XII)

dans laquelle A, $R_4$, $R_5$, Y, n et m ont les significations qui ont été données ci-dessus dans cette même revendication et B représente un radical —(—$CH_2$—$)_r$— ou un radical —CONH—(—$CH_2$—$)_r$—NHCO—, dans lesquels r représente un nombre de 2 à 8, ou B représente un alcénylène en $C_4$-$C_8$, un xylylène ou un bitolylène, ou $R_2$ représente un radical —$SO_2R_{14}$ ou —$OR_{15}$ dans lesquels $R_{14}$ désigne un alkyle en $C_1$-$C_4$, un p-tolyle ou un phényle et $R_{15}$ un alkyle en $C_1$-$C_4$, un benzyle ou un radical L'—CO— dans lequel L' a la signification qui a été donnée pour L dans cette même revendication, et

Y représente —O— ou un radical —$N(R_{16})$— dans lequel $R_{16}$ représente l'hydrogène, un alkyle en $C_1$-$C_{12}$, un cyclohexyle, un alcoxyalkyle en $C_3$-$C_7$ ou un radical répondant à la formule XIII :

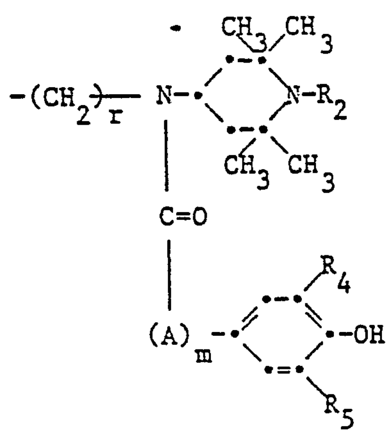

(XIII)

dans laquelle r, m, A, $R_2$, $R_4$ et $R_5$ ont les significations qui leur ont déjà été données dans cette même revendication.

3. Matière d'enregistrement pour photographie en couleur selon la revendication 1, matière caractérisée en ce qu'elle contient, comme stabilisant à la lumière, au moins un composé polyalkylpipéridinique répondant à la formule XIV :

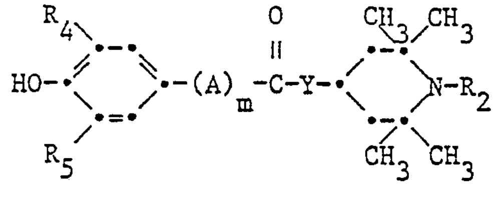

(XIV)

dans laquelle m est égal à 0 ou à 1,

A représente un radical méthylène ou éthylène,

$R_4$ et $R_5$ représentent chacun, indépendamment l'un de l'autre, un radical méthyle ou tert-butyle,

Y représente —O— ou un radical —N($R_{16}$)— dans lequel $R_{16}$ représente l'hydrogène ou un alkyle en $C_1$-$C_8$, et

$R_2$ représente un radical hydroxy, méthyle, allyle, benzyle, hydroxy-2 éthyle, acétyle, acryloyle, méthoxy ou acétoxy ou un radical répondant à la formule XV :

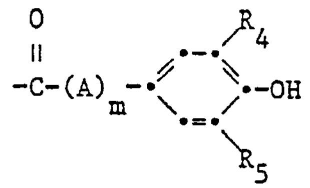

(XV)

dans laquelle m, A, $R_4$ et $R_5$ ont les significations qui ont été données dans cette revendication, ou $R_2$ représente un radical de formule IV dans lequel $R_{10}$ représente un alkyle en $C_1$-$C_8$, un cyclohexyle ou un phényle et $R_{11}$ l'hydrogène, un alkyle en $C_1$-$C_8$ ou un cyclohexyle.

4. Matière d'enregistrement pour photographie en couleurs selon la revendication 1, caractérisée en ce qu'elle contient, comme stabilisant à la lumière, au moins un composé polyalkyl-pipéridinique répondant à la formule XIV :

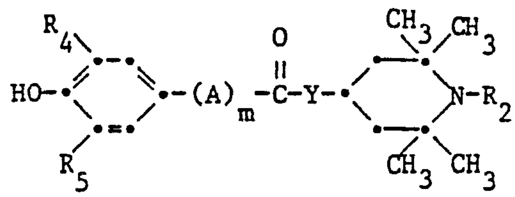

(XIV)

dans laquelle $R_2$ représente un radical de formule :

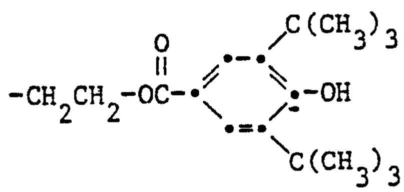

et m, A, $R_4$, $R_5$ et Y ont les significations données à la revendication 3.

5. Matière d'enregistrement pour photographie en couleurs selon la revendication 1, caractérisée en ce qu'elle contient, comme stabilisant à la lumière, au moins un composé polyalkyl-pipéridinique répondant à la formule XIV :

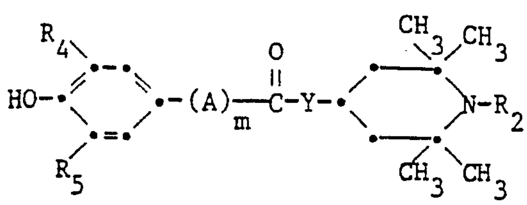

(XIV)

dans laquelle A représente un radical —$CH_2$—C(E)(G)— dans lequel E désigne un radical cyano, —$COCH_3$ ou —$COOCH_3$ et G l'hydrogène ou un radical répondant à l'une des deux formules suivantes :

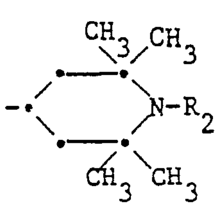

et

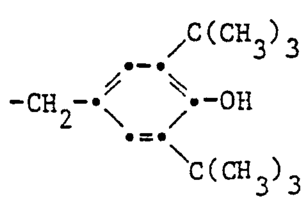

ou encore E et G forment ensemble un radical $-CO-(-CH_2)_4-$, $R_2$ représente un radical —CHO, acétyle ou acryloyle, et m, $R_4$, $R_5$ et Y ont les significations données à la revendication 3.

6. Matière d'enregistrement pour photographie en couleurs selon la revendication 1, caractérisée en ce qu'elle contient le stabilisant à la lumière associé à des copulateurs pour bleu vert, pourpre et jaune.

7. Matière d'enregistrement pour photographie en couleurs selon la revendication 1, caractérisée en ce qu'elle contient le stabilisant à la lumière associé à des absorbeurs de rayons ultra-violets.

8. Matière d'enregistrement pour photographie en couleurs selon la revendication 7, caractérisée en ce que les absorbeurs de rayons ultra-violets sont des composés du type de la benzophénone, de l'acrylonitrile, de la thiazolidone, du benzotriazole, de l'oxazole, du thiazole ou de l'imidazole.

9. Matière d'enregistrement pour photographie en couleurs selon la revendication 1, caractérisée en ce qu'elle contient le stabilisant à la lumière associé à des copulateurs pour bleu vert, pourpre et jaune et à des absorbeurs de rayons ultra-violets, dans la même couche.

10. Matière d'enregistrement pour photographie en couleurs selon la revendication 1, caractérisée en ce qu'elle contient de 1 à 2 000 mg du stabilisant à la lumière par $m^2$ de la couche au sein de laquelle celui-ci est incorporé.

11. Procédé pour réaliser des images photographiques en couleurs, procédé selon lequel on expose à la lumière une matière d'enregistrement pour photographie en couleurs selon la revendication 1, et on développe les couleurs.

12. Images photographiques en couleurs qui ont été obtenues par le procédé de la revendication 11.

13. Composés de formule I dans lesquels Y représente un radical $-N(R_{16})-$, n représente le nombre 1, et $R_1$, $R_2$, $R_3$, $R_4$, $R_5$, $R_{16}$, A et m ont les significations données à la revendication 1.

14. Composés de formule X selon la revendication 13, caractérisés en ce que Y représente un radical $-N(R_{16})-$, n représente le nombre 1, et $R_2$, $R_4$, $R_5$, $R_{16}$, A et m ont les significations données à la revendication 2.

15. Composés de formule XIV selon la revendication 13, caractérisés en ce que Y représente un radical $-N(R_{16})-$ et $R_2$, $R_4$, $R_5$, $R_{16}$, A et m ont les significations données à la revendication 3.